# EUROPEAN PATENT APPLICATION

(11) **EP 4 417 132 A1**
(43) Date of publication of application: **21.08.2024**
(21) Application number: 22881013.1
(22) Date of filing: 11.10.2022
(51) Int. Cl.: A61B 6/03, A61B 5/022, A61B 5/0295, A61B 5/055, A61B 6/00

(54) **DIAGNOSIS ASSISTING PROGRAM**

(30) Priority: 11.10.2021 JP 2021166909; 11.03.2022 JP 2022038693
(71) Applicant: Radwisp Pte. Ltd., The Central 059819 (SG); Mediott Co., Ltd., Tokyo 162-0803 (JP)
(72) Inventor: ABE, Takehiko, 059819 Singapore (SG); YOSHIDA, Norifumi, 059819 Singapore (SG)
(74) Representative: Isern Patentes y Marcas S.L.
(86) International application number: PCT/JP2022/037892
(87) International publication number: WO 2023/063318

(57) **Abstract**

There is provided a diagnostic support program capable of displaying movement of an organ. Provided is the diagnostic support program that analyzes images of a human organ and displays analysis results, the program causing a computer to execute a process comprising processing of acquiring a plurality of frame images; processing of calculating a frequency characterizing a state of the organ based on each of the frame images; processing of calculating a phase difference between a waveform in a previously acquired organ model and a waveform corresponding to the calculated frequency; and processing of outputting a signal indicating the phase difference.

## Description

### TECHNICAL FIELD

The present invention relates to a diagnostic support program that analyzes images of a human organ and displays analysis results.

### BACKGROUND ART

In recent years, the morality rate due to heart diseases has been on the rise, and the necessity of useful and simple diagnostic technology has been highly increasing. MRI diagnostic technology has been making rapid progress, and the importance of MRI has been rising in an image diagnosis of a cardiac area since it becomes possible to conduct various cardiac examinations in a short time. Regarding "cardiac MRI", examinations such as "cine MRI", "perfusion", "delayed contrast-enhanced method", and "BB (black blood) method" are conducted. Especially, cine MRI that has no limit on the examination range is able to observe arbitrary cross sections and is highly reproducible, compared to an ultrasonic examination, SPECT examination, or the like. Therefore, imaging is performed generally in many medical facilities. As for cine MRI, for example, data collection of "approximately 10 slices / 20 phases" is performed for an entire left ventricle by using an electrocardiogram gating method. Recently, a high contrast between blood and myocardium can be obtained by using "Steady State method". Further, in "cardiac function analysis", the necessity of evaluation of cardiac functions by MRI is increasing because accurate values can be obtained as compared with CT, LVG (left ventriculography), and SPECT.

As described above, cardiac MRI is clinically useful, and in particular, imaging is performed in cine MRI in many medical facilities, but the images were rarely analyzed by using software. The reason for this is said to be that the software used for conventional cardiac function analysis requires complicated operations to extract and correct the contours of the inner and outer sides of myocardial membranes. Moreover, the reproducibility of the analysis results has also been an issue, since the contour tracing of the inner and outer sides of myocardial membranes is easily influenced by an individual operator. Furthermore, in the situation where cardiac MRI has become more widespread and more medical facilities are using MRI systems provided by multiple manufacturers, the names of the sequences used by each company differ, making it difficult to easily handle the data.

In order to solve such problems, and to reduce the labor of tracing complicated myocardial contours, software that improves the accuracy thereof and automatically performs processing of interpolation to enable the correction work to be reduced even if an unintended tracing is made, has been provided. In this software, less stressful image observation is enabled by displaying images side by side on the viewer for MRI cardiac function analysis and by flicking operation.

### PRIOR ART DOCUMENT

### NON-PATENT DOCUMENT

Non-Patent Document 1: https://www.zio.co.jp/ziostation2/

### SUMMARY OF THE INVENTION

### PROBLEMS TO BE SOLVED BY THE INVENTION

However, it is not easy for a doctor to grasp the pathological condition only by simply displaying MRI images side by side as in the technique described in Non-Patent Document 1. Consequently, it is desirable to display images in accordance with the state of heart. That is, it is desirable to grasp a heart of a human body as a subject, and to display images showing an actual movement, based on a tendency of a change in a waveform or a frequency of a heart, or in an image thereof.

Further, according to movement of an abnormal organ with respect to movement of a normal organ, it is gradually understood that a phase in a cycle thereof is shifted, but no technique of displaying images of the organ has been put to practical use, based on this fact.

The present invention has been made in view of such a situation and has an object to provide a diagnostic support program capable of displaying movement of an organ. More specifically, it has an object to generate images that assist a diagnosis by calculating numerical values that assist a diagnosis by digitizing the concordance rate, phase shift, or another non-concordance rate for the waveform and Hz already acquired for new target data to be measured and further by imaging these numerical values.

### MEANS TO SOLVE THE PROBLEMS

(1) In order to achieve the above-described object, the present application has taken the following steps as described below. That is, it is a feature that the diagnostic support program according to one aspect of the present invention is a diagnostic support program that analyzes images of a human organ and displays analysis results, the program causing a computer to execute a process comprising processing of acquiring a plurality of frame images; processing of calculating a frequency characterizing a state of the organ based on each of the frame images; processing of calculating a phase difference between a waveform in a previously acquired organ model and a waveform corresponding to the calculated frequency; and processing of outputting a signal indicating the phase difference.
(2) Further, it is a feature that in the diagnostic support program according to one aspect of the present invention, the organ model is determined by an average value of pixel values within each divided area, by dividing the images of the organ thereinto
(3) Further, it is a feature that in the diagnostic support program according to one aspect of the present invention, the images of the organ are divided thereinto using a Voronoi tessellation method.
(4) Further, it is a feature that in the diagnostic support program according to one aspect of the present invention, a lung field area is divided thereinto according to a change rate of a lung volume, when the organ is a lung.
(5) Further, it is a feature that the diagnostic support program according to one aspect of the present invention is a diagnostic support program that analyzes images of a human organ and displays analysis results, the program causing a computer to execute a process comprising processing of acquiring a plurality of frame images; processing of extracting pixel values at specific points included in the images of the organ; processing of calculating a temporal change of the extracted pixel values; processing of extracting at least one frequency signal from the temporal change of the pixel values; and processing of outputting the extracted frequency signal as a signal at the specific points.
(6) Further, it is a feature that the diagnostic support program according to one aspect of the present invention is a diagnostic support program that analyzes images of a human organ and displays analysis results, the program causing a computer to execute a process comprising processing of acquiring the images of the organ, that are imaged from multiple directions under a plurality of imaging conditions; processing of extracting pixel values at specific points included in the image of the organ; processing of calculating a temporal change of the extracted pixel values; processing of extracting at least one frequency signal from the temporal change of the pixel values; processing of outputting the extracted frequency signal as a signal at the specific points; and processing of preparing a cross-sectional diagram of all or part of the organ, using the output signal at the specific points.
(7) Further, it is a feature that in the diagnostic support program according to one aspect of the present invention, the images of the organ are divided into certain areas including the specific points, using a Voronoi tessellation method.
(8) Further, it is a feature that the diagnostic support program according to one aspect of the present invention is a diagnostic support program that analyzes images of a human organ and displays analysis results, the program causing a computer to execute a process comprising processing of acquiring the signal output from the diagnostic support program according to any one of (1) to (7); processing of making AI (Artificial Intelligence) learn the signal indicating a normal organ or the signal indicating an abnormal organ according to the acquired signal; and processing of recording learning results obtained by the Al.
(9) Further, it is a feature that the diagnostic support program according to one aspect of the present invention is the program causing the computer to execute the process further comprising processing of acquiring the plurality of flame images; and processing of specifying the organ from the acquired flame images, and outputting a ratio of an abnormal value by comparing the specified organ with the learning results.
(10) Further, it is a feature that the diagnostic support program according to one aspect of the present invention is the program causing the computer to execute the process further comprising processing of outputting a phase difference between a waveform representing a cyclic motion of the normal organ and a waveform representing a cyclic motion of the abnormal organ.
(11) Further, it is a feature that the diagnostic support program according to one aspect of the present invention is the program causing the computer to execute the process further comprising processing of adding/subtracting a signal in one region exhibiting different permeability therefrom to/from a signal in the other region, according to each of the plurality of flame images.
(12) Further, it is a feature that the diagnostic support program according to one aspect of the present invention is the program causing the computer to execute the process further comprising processing of converting a waveform of a cyclic signal into a trigonometric function; and processing of outputting a signal indicating the waveform converted into the trigonometric function.
(13) Further, it is a feature that the diagnostic support program according to one aspect of the present invention is a diagnostic support program that analyzes images of a human organ and displays analysis results, the program causing a computer to execute a process comprising processing of acquiring a plurality of frame images imaged by applying a cyclic signal to a human body; processing of Fourier-transforming a change in pixel value in a specific area in each of the frame images; processing of extracting a spectrum in a fixed band including a spectrum corresponding to a frequency of the signal applied to the human body, out of a spectrum obtained after the Fourier-transforming; processing of subjecting the spectrum extracted from the fixed band to inverse Fourier transform; and processing of displaying each of the images after performing the inverse Fourier transform, on a display.
(14) Further, it is a feature that the diagnostic support program according to one aspect of the present invention is a diagnostic support program that analyzes images of a human organ and displays analysis results, the program causing a computer to execute a process comprising processing of acquiring a plurality of frame images imaged by applying a cyclic signal to a human body; processing of calculating a change rate of a pixel value in a specific area in each of the frame images; processing of extracting only an area for which a tunable rate is within a predetermined fixed range, using the tunable rate that is a value of a ratio of the change rate of the pixel value in the specific area to a cyclic change rate of the signal applied to the human body; and processing of displaying each of the images including the extracted area, on a display.
(15) Further, it is a feature that the diagnostic support program according to one aspect of the present invention is a diagnostic support program that analyzes images of a human organ and displays analysis results, the program causing a computer to execute a process comprising processing of acquiring a plurality of frame images; processing of calculating a function representing a frequency or a waveform of lung blood flow, based on each of the frame images; processing of calculating the function representing the waveform of the lung blood pressure, based on the function representing the calculated frequency and waveform of the lung blood flow, and information indicating size of lung blood vessels; and processing of estimating lung blood pressure from the calculated waveform of the lung blood pressure.
(16) Further, it is a feature that the diagnostic support program according to one aspect of the present invention is the program causing the computer to execute the process further comprising processing of acquiring the plurality of frame images; and processing of specifying the organ and blood flow flowing through the organ from the acquired flame images, and outputting a feature amount of the blood flow in the organ by comparing the specified organ and blood flow with the recorded learning results.
(17) Further, it is a feature that the diagnostic support program according to one aspect of the present invention is the program causing the computer to execute the process further comprising processing of outputting a feature amount of blood flow in main blood vessels of the lung, or blood flow in capillaries and peripheral pulmonary vessels of the lung.
(18) Further, it is a feature that the diagnostic support program according to one aspect of the present invention is the program causing the computer to execute the process further comprising processing of comparing movement during lung respiration with movement of lung blood flow, and outputting a feature amount indicating a linkage between both the movements.
(19) Further, it is a feature that the diagnostic support program according to one aspect of the present invention is the program causing the computer to execute the process further comprising processing of acquiring the plurality of frame images; and processing of specifying the lung field area from the acquired frame images, comparing a wave indicating movement in the specified lung field area with a reference wave, and outputting a feature amount indicating a linkage between the waves.
(20) Further, it is a feature that in the diagnostic support program according to one aspect of the present invention, the reference wave is a wave indicating a respiratory cycle.
(21) Further, it is a feature that the diagnostic support program according to one aspect of the present invention is the program causing the computer to execute the process further comprising processing of calculating a maximum value of pixel value; and processing of acquiring the waveform, based on a signal after the calculated maximum value.
(22) Further, it is a feature that in the diagnostic support program according to one aspect of the present invention, Fourier-transforming processing is carried out by inputting data having periodicity, and inverse Fourier transform processing is carried out by performing filtering processing by which a specific frequency is extracted.
(23) Further, it is a feature that in the diagnostic support program according to one aspect of the present invention, a plurality of waveforms are superposed, and a phase peak in one cycle of any waveform is detected to calculate a phase difference of any other waveform.
(24) Further, it is a feature that in the diagnostic support program according to one aspect of the present invention, a lung image is divided into a plurality of areas, and an average and a distribution of intensity values in the respective areas are calculated to obtain a correlation with a count value according to lung scintigraphy in between.
(25) Further, it is a feature that in the diagnostic support program according to one aspect of the present invention, a basic waveform is generated by superposing a plurality of original waveforms obtained from the images, respectively; and the original waveforms are subjected to setting of a band width thereof or weighting, while generating a master waveform based on the basic waveform to generate a waveform corresponding to the organ in each of the images.

### EFFECT OF THE INVENTION

According to one aspect of the present invention, it becomes possible to grasp an organ of a human body as a subject, and to display images each showing actual movement, based on a tendency of a change in a waveform or a frequency of an organ, or in an image thereof.

### BRIEF DESCRIPTION OF THE DRAWINGS

FIG. 1 is a diagram showing an outline configuration of a diagnostic support system according to the present embodiment.
FIG. 2A is a diagram showing a sectional view of a heart.
FIG. 2B is a diagram showing a sectional view of a heart.
FIG. 2C is a diagram showing a sectional view of a heart.
FIG. 2D is a diagram showing an example of Voronoi tessellation.
FIG. 3A is a diagram showing a change in "intensity" of a specific block and a result obtained by performing Fourier analysis thereof.
FIG. 3B is a diagram showing a Fourier transform result obtained by extracting a frequency component close to a heartbeat and a change in "intensity" of the frequency component close to the heartbeat, that is obtained by performing inverse Fourier transform on this.
FIG. 3C is a diagram showing an example of extracting a certain fixed band out of a spectrum obtained after Fourier-transforming.
FIG. 4 is a flowchart showing an outline of image processing according to the present embodiment.
FIG. 5 is a flowchart showing an outline of image processing according to the present embodiment.
FIG. 6 is a flowchart showing an outline of image processing according to the present embodiment.
FIG. 7A is a schematic diagram showing a left lung of a human body from the front.
FIG. 7B is a schematic diagram showing a left lung of a human body from the left side face.
FIG. 8A is a schematic diagram showing a left lung of a human body from the front.
FIG. 8B is a schematic diagram showing a left lung of a human body from the left side face.
FIG. 9A is a schematic diagram showing a left lung of a human body from the front.
FIG. 9B is a schematic diagram showing a left lung of a human body from the left side face.
FIG. 10A is a schematic diagram showing a left lung of a human body from the front.
FIG. 10B is a schematic diagram showing a left lung of a human body from the left side face.
FIG. 11 is a diagram showing one example of a method of detecting a lung field according to the present invention.
FIG. 12 is a diagram showing a state where blood flow of a lung does not become uniform.
FIG. 13 is a diagram showing a state where blood flow of a lung does not become uniform.
FIG. 14 is a diagram showing a situation where an analysis range of a lung is divided into quarters.
FIG. 15 is a diagram showing a result of approximating a change in pixel value of a lung to sine waves.
FIG. 16 is a diagram showing a result of approximating a change in pixel value of a lung to sine waves.
FIG. 17A is a diagram showing a correlation between FEV (Forced Expiratory Volume in one second) and a phase shift.
FIG. 17B is an example of showing a concept of phase difference.
FIG. 18 is a diagram showing a respiratory condition.
FIG. 19 is a diagram showing a change in lung blood flow within one heartbeat of a normal lung.
FIG. 20 is a diagram showing a change in lung blood flow within one heartbeat of an abnormal lung.
FIG. 21 is a diagram showing a normal lung, and a lung of PAH {pulmonary arterial hyper tension: pulmonary artery pulmonary hyper tension (including lung arteriosclerosis)}.
FIG. 22 is a diagram showing an image of a normal lung.
FIG. 23 is a diagram displaying a normal lung and a lung of COPD.
FIG. 24 is a diagram showing a stereoscopic structure on pulmonary apex-diaphragm side.
FIG. 25 is a diagram showing a situation of peripheral lung field.
FIG. 26 is a diagram showing shallow respiration and deep respiration of a normal lung.
FIG. 27 is a diagram showing a lung of a cardiac failure patient.
FIG. 28 is a diagram showing a lung of a cardiac failure patient.
FIG. 29A is a diagram showing a low resolution difference image obtained by aggregating change in luminance value by enlarging a block size.
FIG. 29B is a diagram showing a high resolution difference image obtained by aggregating change in luminance value by reducing a block size.
FIG. 29C is a diagram showing image examples of MBDP and ABDP.
FIG. 29D is a diagram showing a situation where an image of a lung is divided into 6 areas.
FIG. 30A is a diagram showing lung blood flow during one heartbeat of a heart for every frame.
FIG. 30B is a diagram showing measurement result examples of MPA (Main Pulmonary Artery), DPA (Distal Pulmonary Artery), and DPV (Distal Pulmonary Vein).
FIG. 30C is a diagram showing signal intensity according to an ultrasonic image of an atrium.
FIG. 30D is a diagram showing signal intensity according to an ultrasonic image of an atrium.
FIG. 30E is a diagram showing movement of a respiration wave.
FIG. 30F is a diagram showing a stereoscopic structure and a cross-section by chest XP images.
FIG. 30G is a diagram showing a normal lung and a patient's lung.
FIG. 30H is a diagram showing a patient's lung.
FIG. 30I is a diagram showing a patient's lung.
FIG. 30J is a diagram showing comparison between shallow respiration and deep respiration.
FIG. 30K is a diagram showing a state where waves of diaphragm or the like are shifted with respect to the original wave.
FIG. 30L is a diagram showing an image of a pneumonia patient.
FIG. 30M is a diagram showing one example of electrocardiogram.
FIG. 31A is a diagram showing an example of contrasting between a waveform of aorta blood flow quantity and a waveform of ventricular volume.
FIG. 31B is a diagram showing an example of two lung images.
FIG. 31C is a diagram showing an example obtained by taking a difference between two lung images, and setting a fixed threshold value to visualize the difference.

### DETAILED DESCRIPTION OF EMBODIMENTS

The present inventors focused on the fact that technology for visualizing a movement of organs (for example, myocardium of a heart) has not been put into practical use conventionally, and found that it becomes possible to support a diagnosis of a doctor by expressing not only deviation of a movement of organs but also places where they are not moving, and came up with the present invention.

That is, it is a feature that the diagnostic support program according to one aspect of the present invention is a diagnostic support program that analyzes images of a human organ and displays analysis results, the program causing a computer to execute a process comprising processing of acquiring a plurality of frame images; processing of calculating a frequency characterizing a state of the organ based on each of the frame images; processing of calculating a phase difference between a waveform in a previously acquired organ model and a waveform corresponding to the calculated frequency; and processing of outputting a signal indicating the phase difference.

In this manner, a diagnosis that required a lot of skill can be simplified, and moving images can be qualitatively represented to give objectivity to displayed contents. Although a heart is used for explanation as an example of an organ in the present specification, it goes without saying that the present invention is not limited to a heart but can also be applied to various organs such as lungs and blood vessels.

In the invention according to the present application, it is possible to be determined in comparison with previous images of a subject; with those obtained by averaging a range to be analyzed and the entire organ; with normal images; and with age samples, by adjusting (standardizing) movement of the organ so as to be considered constant.

### [Basic concept]

The basic concept in the present invention is described. In the present invention, as to a cyclic change that characterizes a state of an organ in a human body, for example, a cross-sectional area, a surface area and volume of a heart, with respect to a movement caught in a repetitive manner in a fixed cycle, a fixed repetition or a fixed movement (routine) on a time axis in the entire or certain partial range is caught as a wave, and measured. For measurement results of the wave, (a) a form of a wave itself or (b) wave intervals (frequency: Hz) is used.

For example, as to images of a heart, waves that are linked similarly during the same period of time may exist. For example, in the case of a heartbeat, the following approximation can be conceptualized. (Average of a change in density in a rough range) ≈ (Heartbeat) ≈ (Change of a heart) ≈ (Electrocardiogram) ≈ (Change in a surface area and volume of a heart)

In the present invention, for example, with respect to a heart, it is possible to analyze by focusing on deformation of regional ventricular walls (wall thickness dilatation, wall thickness contraction), and it is possible to catch and to cyclically represent the systolic thickness, diastolic thickness, and a wall movement (inner and outer membranes). Furthermore, as to a heart, it is possible to catch and to cyclically represent "relative wall thickness (RWT)", which is calculated from "(2 x posterior wall thickness) / left ventricular end-diastolic diameter)", and "ejection fraction", which is calculated from "(end-diastolic volume - end-systolic volume) / (end-diastolic volume)".

In the present invention, it is made possible to extract an image with higher accuracy, by using any of these data or data obtained by using those in combination. In this case, calculations may be sometimes interactively carried out many times. At this time, the artifact with respect to the results is eliminated again, and extraction of the function is carried out by extracting from the new data extraction waveform, the data waveform to be the first base, another waveform of modality or the like, the periphery, and the waveform of plural times. In this case, the number of times may be either once or plural times.

Herein, when producing the base data, mutual component extractions are made up for each other by a plurality of modalities (for example, a certain density, a change amount constituted by volumetry, heart movement, and so forth), or by plural times of waveform measurement for a heartbeat and so forth, thereby improving accuracy. By doing this, it becomes possible to reduce the artifact and to improve the accuracy based on a certain fixed prediction of a line or the like.

Herein, "density" is translated as "mitsudo", but in an image, it means an "absorption value" of pixels in a specific area. For example, in the case of CT, air, bones and water are used as "-1000", "1000", and "0", respectively.

In the present specification, "density" and "intensity" are distinctively used. As described above, "density" means an absorption value and exhibits high air permeability in original images of XP and XP moving images, and air, water and bones are to be displayed as "-1000", "0" and "1000", respectively, by digitizing a portion exhibiting the high permeability as being white. On the other hand, "intensity" is one relatively changed from "density", for example, one displayed via "conversion" into a degree of density and a signal width by being normalized. That is, "intensity" is a relative value of light-dark, an emphasis degree, and so forth. It is represented as "density" or "a change in density (Δ density)" during directly dealing with the absorption value of an XP image. Then, this is converted thereinto as described above, for image expression reasons, and represented as "intensity". For example, "intensity" is given in a case where color displaying to 256-step gray scales of from 0 to 255 is carried out. Such a terminology distinction is applicable to the case of XP or CT.

On the other hand, in the case of MRI, even though air, water and bones are attempted to be set as "-1000", "0" and "1000", respectively, there is a situation in which the values are largely changed due to pixel values of MRI, types of measuring machines, a person's physical conditions at the time of measurement, build of a body, and measuring time; and how to acquire signals of MRI such as T1 emphasis images and so forth also varies by a facility thereof and the types of measuring machines, thereby being unable to be fixed. Accordingly, in the case of MRI, no definition of "density" can be applied thereto as with the case of XP and CT. Therefore, MRI deals with relative values from a stage of initial extraction, thereby expressing as "intensity" from the beginning. Then, the signals for processing are also "intensity".

From those described above, it becomes possible to obtain master data. For the above-described master data, a new target desired to be measured is extracted in a certain fixed width and range of a waveform and Hz of a wave of the above-described master data. For example, extraction is performed only for a heartbeat, or in the width or range as a frame of a degree of blood vessel extraction. In addition, this waveform and the width of Hz are relatively and collectively determined based on statistics by using the waveform element in another function, artifacts such as noise and so forth, the waveform of another modality deemed to have another tunability, reproducibility performed plural times, and so forth. Then, adjustment and experience are required therein (it is also possible to apply machine learning thereto). This is because while the width and range are extended, the element of another function begins to enter, and if they are too narrow, the element of the function itself is eliminated, and thus the range needs to be adjusted. For example, in the case of presence of data of plural times, it is easy to specify the range, Hz, the concordance width in measurement, and so forth. Further, fluctuations in axis, width, range, and Hz due to the mutual component extractions and the width can be estimated. That is, by plural times of superimposition, the axis setting of Hz is averaged and the optimum range of each of the axis, width, range, and Hz is calculated via variance. At this time, there is a case where Hz (noise) of another behavior is extracted, and if a wave thereof exists, the degree in which no wave is included in the case of presence of the wave is also relatively measured.

Next, for the new target data to be measured, a concordance rate and other non-concordance rates with already caught waveform and Hz are quantified to calculate numerical values that assist a diagnosis. For example, it can be applied to a diagnostic assist instrument by measuring a waveform concordance rate of disease of a master and calculating the disease waveform concordance rate along with noise exclusion of a pulsimeter or auscultation. In the present specification, a case where a tunable concordance rate of pixel values can be used is described.

### [With regard to a tunable concordance rate]

In the present specification, a tendency of an image change is explained as a tunable concordance rate. For example, a myocardial area is detected and divided into a plurality of block areas to calculate average density (pixel value x) of the block areas in each frame image. Then, a ratio (x') of an average pixel value of block areas in each frame image to a change width (0% to 100%) from the minimum value to the maximum value of average density (pixel value x) is calculated. On the other hand, by using a ratio value (x'/y') of a ratio (y') of a change (y) in myocardium of each frame image to a change width (0% to 100%) from at the minimum position to at the maximum position of the myocardium, only block areas for which the ratio value (x'/y') is within a predetermined fixed range are extracted.

Herein, the case where y'=x' or y=ax (a represents a numerical value of an amplitude of myocardium, or a coefficient of a numerical value of density) means complete concordance. However, it does not mean that only the case of the complete concordance indicates a meaningful value, and a value having a certain fixed width should be extracted. Thus, according to one aspect of the present invention, a fixed width is determined using logarithms (log), as described below. That is, when calculated at a ratio (%) in the case where y=x, complete concordance of tunability is "log Y'/x'=0". Further, when extracting one in which a range of a tunable concordance rate is narrow or a (numerically narrow) range, for example, it is determined as "log Y'/x'=-0.05 to +0.05" in the range that is close to 0, and when being one in which a range of a tunable concordance rate is wide or a (numerically wide) range, for example, it is determined as "log Y'/x'=-0.5 to +0.5" in the range that is close to 0. As this range is narrower, and the numerical value that is concordant within the range is also higher, the concordance rate can be said to be higher. When counting the number by determining this ratio value for each pixel of the pixels, a normal distribution in which the case of complete concordance is taken as a peak is obtained in the case of healthy persons. In contrast, in the case of those having disease, a distribution of this ratio value is to be lost. In addition, as described above, the method of determining a width using logarithms is only one example, and the present invention is not limited thereto.

That is, the present invention is one performing "image extraction" as the following: (average of a change in density in a rough range) ≈ (a heartbeat) ≈ (a change in a heart) ≈ (an electrocardiogram) ≈ (a change in a surface area and volume of a heart), and is also applicable to methods other than the method of using logarithms. It becomes possible to display a frequency tunable image via such a method.

In the case of blood vessels, as to a series of changes of density {x (one waveform of myocardium)} produced by responding to a series of contractions of a heart (y), a slight time delay (a change in phase) is, as is, present, thereby being displayed as y=a'(x-t). In the case of complete concordance, since t=0, y=x or y=a'x. Here, when extracting one in which a range of a tunable concordance rate is narrow or a (numerically narrow) range, for example, it is determined as "log y'/x'=-0.05 to +0.05" in the range that is close to 0, and when being one in which a range of a tunable concordance rate is wide or a (numerically wide) range, for example, it is determined as "log y'/x'=-0.5 to +0.5" in the range that is close to 0. As this range is narrower, and the numerical value that is concordant within the range is also higher, the concordance rate can be said to be higher.

In the case of other blood vessels, the above-described "portion responding to a heart" is excluded, and density on the center side that is plotted from pulmonary hilum can be used. The case of peripheral blood vessels may be also similarly taken care of.

Further, the present invention may also be applied to a circulatory system. For example, a change in density of a heart is directly associated with a change in density of a blood flow to a pulmonary hilum portion - a peripheral lung field, and a change in a series of changes in density of the heart and a change in density of the pulmonary hilum portion are subjected to a type of conversion, and transmitted, as is, thereto. It appears that this is produced by obtaining a slight phase difference from the relationship between the change in density of the heart and the change in density of the pulmonary hilum portion. Further, a change in density of the pulmonary hilum portion or the like is associated, as is, with a change in density of a lung field to a blood flow, and thus it is possible to express tunability by one (concordance rate relationship in y ≈ x) reflected with an as-is rate. Further, it appears that as to a cervical blood vessel system, a change in density plotted at central heart blood vessels on the periphery thereof is directly associated therewith, or also associated therewith accompanying a slight phase in a similar manner. Then, when the density varies depending on the background, and is propagated, it becomes possible to be considered as a tunable concordance rate so as to propagate the situation of a change in density.

Herein, regarding each of a change amount in one image and a change rate in one image, for being displayed as a relative value (Standard Differential Signal Density/intensity) when a change amount from density of a heart is set to 1, the change amount and the change rate can be extracted for each of (1) as to a different image for each image, an image when 1 is set to each one (general assumption), (2) as to a different image for each one, a ratio when the a heartbeat obtained by adding density (a change amount and a change rate) thereto is set to 1, and (3) as to carrying out imaging plural times, the ratio obtained as a total amount of density while taking each heartbeat as being set to 1.

Further, in the case of 3D of MR or the like, as to a value (when it is set to 1 at this time) obtained by summing intensity (in the case of MR) or density (in the case of CT) of a heartbeat, the difference of its intensity or density can be converted into "peak flow volume data" of the heartbeat (during rest, or even during a load), and as to this value, an actual measurement amount of movement and a movement rate of a heart can be converted thereinto by finding a ratio of intensity or density when calculating "3D × time" with at least MRI, CT or the like. Similarly, it also becomes possible that a distribution in "capillary phase" of "flow" in a lung field presents an estimation value of being converted into a distribution of a lung blood flow peripheral amount, or volume by inputting one time cardiac output.

That is, (average of a change in density in a rough range) ≈ (a heartbeat) ≈ (a change in a heart) ≈ (an electrocardiogram) ≈ (a change in a surface area and volume of a heart) is satisfied, and when taking out only a change amount of one with 10 % or 20 %, an estimation value is possible to be calculated by calculating (the whole number of those) × (a change amount of the time).

Then, for the new target data to be measured, images that assist a diagnosis are calculated, by imaging the concordance rate or another non-concordance rate for the waveform and Hz that are caught beforehand. For example, the differences between normal swallowing and a patient's swallowing are visualized, and the differences between movements in which the patient has been making so far and movements in which the patient is currently making are shown. For example, changes and differences in the way of walking and in swinging are included.

The extracted change amount is visualized and extracted onto an image. This is cardiac function analysis and blood vessel (blood flow) analysis as described below. Then, a change rate of myocardium is visualized. At this time, there are some cases where the artifact with respect to the results is eliminated again and extraction of the function is carried out by extracting from the new data extraction waveform, the data waveform to be the first base, another waveform of modality or the like, the periphery, and the waveform of plural times. The method of eliminating the artifact is described later.

Further, there are some cases where the feature amount is grasped even from those from which change components extracted from other than those extracted as described above are excluded. For example, when grasping a movement of the abdominal intestinal tract, an attempt is made to extract the movement of the abdominal intestinal tract by excluding the influence of respiration and the influence of blood vessels from the abdomen.

Further, based on the change rate due to the extraction, correction is applied to images that take a certain fixed amount of imaging time (CT, MRI, special radiography, PET/scintigraphy, and the like) to provide clearer and more accurate images. For example, it is useful for ascending aorta cardiac correction, cardiac morphology correction, correction of bronchial blurring, evaluation of the periphery of a thorax, and imaging when the patient is unable to hold his or her breath (that may take several minutes for imaging a patient).

Further, a basic waveform can be obtained by dividing plural waveforms into those in one cycle, and superposing data. A band width (frequency width) of the basic waveform, weighing to the basic waveform, a master waveform component obtained via an average, dispersion or the like of the basic waveform, weighing to a waveform obtained from each of original images, and a band width component are associated with the waveform obtained from the original image to be fitted to a waveform of each of conversion images. Weighing to each conversion image and a width component of logarithmic conversion can be fitted to a waveform of each image-conversion image to be used for calculation during image conversion such as the logarithmic conversion or the like. That is, a basic waveform is generated by superposing a plurality of original waveforms obtained from the images, respectively; and the original waveforms are subjected to setting of a band width thereof or weighting, while generating a master waveform based on the basic waveform to generate a waveform corresponding to the organ in each of the images. Thus, it becomes possible to contribute to a diagnostic support. Further, a width of a tunable concordance rate and a difference for tunable concordance, and a ratio thereof become clear, and thus a fixed band width can be measured. Abnormality can be extracted by forming the band in this manner, and changing a phase to carry out a simulation. In an image as a target, a peak pixel value can be calculated when measuring density at a peak of a phase of one cycle, and it is easy to grasp clear appearance of a phase difference when setting it at the peak as a base. When the phase is shifted in one cycle, part that is large in phase shifting is extracted. A large shifted part can also be extracted for other components each whose cycle is shifted to display those as being abnormal. When being abnormal, not only the waveform but also the amplitude is shifted, and thus it can be clearly extracted. Further, the correlation with "Wave form" is made to be higher, and it can also be used for correction (restoration). For example, in the case of lung blood flow, after generation of a heartbeat, it takes 0.016 seconds to get to lung blood vessels, and the peak is shifted from the generation of heartbeat. Some people may require 0.024 seconds. Thus, it becomes possible to acquire a waveform of lung blood flow from pulsation of a heart. It becomes possible to acquire a peak of the lung blood flow, and a peak of the peripheral blood vessels from change in density. Further, conventionally, when displaying phase shifting of the lung blood flow, there are some cases where the phase shifting is processed by fixing two frames with respect to a center, but in the case of this method, those of blood flow coming out with delay are not observed. In the case of the invention according to the present application, it is possible to display those of the blood flow. In addition, the present invention is not limited to blood flow. That is, the above-described method is also applicable to the other organ, respiration and others.

According to how to search the tunable concordance rate, a simulation is carried out in every phase by including conversion for viewing fixed phase shifting but also phases departing from certain fixed one, and concordance rate data and images are searched to extract abnormal lesion. Similarly, as to the amplitude, a different state is subjected to simulating to depict a concordance image near to "wave form". From this, it becomes possible to be identified from the different concordance rate by giving notice of depiction of abnormality. In addition, when catching a moving phase, it is desirable to use a wide range of frequency. For example, it is designed to be caught in a range between 60 and 80 Hz. According to a so-called "Doppler effect", though the frequency becomes large or small, as a result, phases may often change. In order to catch those, a width (range) needs to be widened.

Further, "tune imaging" of a relative lung field space may be carried out, based on a ratio of the structure of the organ and the space. This means that the entire lung field cannot be imaged all at once, even when the currently available CT is provided with a large portion of 320-row or the like, and thus when being partial imaging, those that could not be measured only from the whole lung field space in such a manner that a regional space that has not been covered so far due to falling outside an imaging range at an end-stage of expiration among the movements is put in an image at the end-stage of expiration; a region put in the image at the end-stage of expiration is made to be outside the imaging at the end-stage of inspiration; and so forth similarly make it possible to prepare "tune imaging" via relative position and relative "volume", and air fluctuation. Further, as to the difference of "CT data", clear "reconstruction" of CT and MRI has been carried out by providing no gap, but it is made possible to collect clear difference data/difference image with respect to more cyclic movement by performing "reconstruction" of signals for the organ moving in conformity with change in "basic data (based on wave form)".

### [With regard to phase shifting]

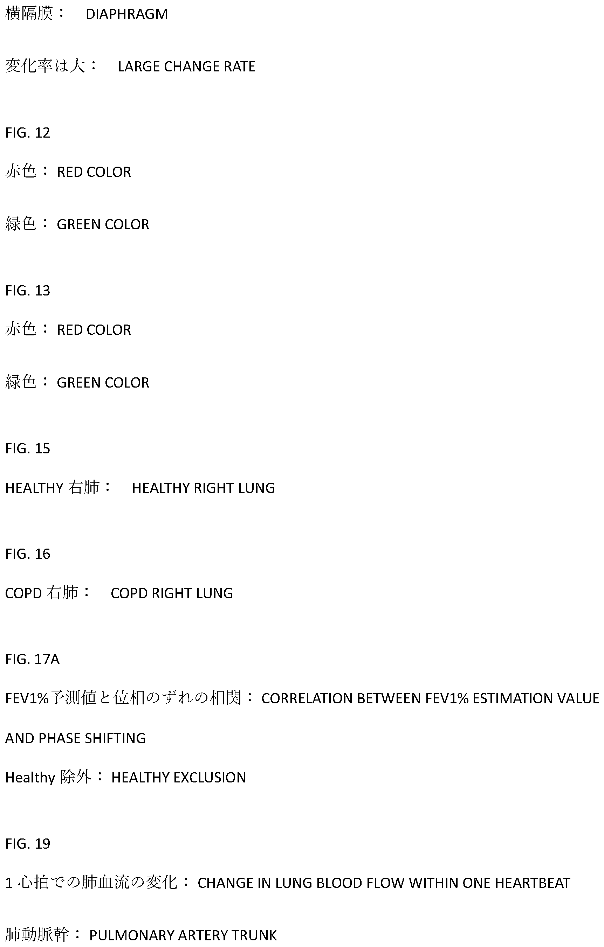

It is known that a sick organ (abnormal organ) exhibits slow reaction in comparison with a histogram of a normal organ. For example, a movement of the abnormal portion of a heart as well as a lung lags behind the movement of the other portion thereof. Then, it becomes possible to display the portion of the abnormal organ with images, by displaying how much shifting is made from a normal frequency. Only the abnormal portion moves slowly as an appearance example of images, and thus a signal value becomes high. Further, initial response is constantly delayed, and shifting of a graph is recognized when being made into a graph. In this manner, according to the abnormal organ, the phase as well as the amplitude is delayed behind the normal organ, and thus the difference (gap) between them is calculated. That is, how much phase of change in pixel value is shifted with a change in normal pixel value as a reference is displayed. Further, the frequency is identical thereto, but there are some cases where it appears as a different waveform. In the present specification, the foregoing relation is called "phase relative imaging" or "phase differential imaging". Further, as shown in FIG. 17B, a phase difference and phase shifting mean a concept concerning not only a phase difference PH1 according to the same wave but also a phase difference PH2 according to a plurality of different waves, but mean the phase difference PH2 according to the plurality of different waves, in the present specification.

For example, a shape of a lung is divided into eight areas, and plotting is carried out by obtaining an average value (intensity) of pixel values included in the areas, respectively. According to normal lungs, phases of movement have a perfect fit with each other, but it is found that according to abnormal lungs, the phases of movement are shifted to each other. The foregoing phase shifting becomes a numerical index determined to be abnormal or normal. Specifically recognized are phenomena such that "a bright line of an image is shifted" or "a phase is shifted from a start time point". Further, when carrying out plotting by obtaining an average value of pixel values in a fixed area, phase shifting can be confirmed.

In the invention according to the present application, a density difference is displayed as a phase difference. How large the phase difference, that is, a movement degree is displayed in the invention according to the present application. The portion in which the movement is small has a small change but the portion in which the movement is large has a large change, thereby resulting in appearance of those on images. That is, a phase difference appears as waveforms, but it appears temporarily delayed as images, it is specifically delayed at a starting time, and being gradually delayed. For example, according to an abnormal lung, an upper lung field exhibits followability thereof, but it is more remarkably delayed toward a middle lung field and a lower lung field. There is also provided no moving portion.

According to the waveform acquired from an organ, a plurality of waves are synthesized. A phase difference is extracted from the waveform and visualized. That is, attention is paid to not only a shifted portion but also being temporarily delayed and being shifted. For example, in "a lung of a patient of pulmonary thrombosis embolism", blood thereof does not uniformly flow, and a phase-shifted waveform appears. Time is shifted, and thus flowing of blood flow is delayed at a shifted portion. Further, shifting appears so as to become larger toward the end-stage from a center of the lung. In the invention according to the present application, it is made possible to display images exhibiting phase difference only using DICOM data. In conventional techniques, it is hard to be dealt with to such an extent as to show a phase difference only using a roentgen image. In the invention according to the present application, the diagnostic support is made possible by visualizing information hidden in the roentgen image.

Further, in terms of fitting a phase to a standard value, for example, an average value or the like, the following method can be employed. For example, a heart is not almost affected by breath-holding, but voluntary and involuntary movements are mixed in respiration. Therefore, as shown in FIG. 18, there are some cases where a phase (the above-described example indicates a breathing-out state, and a plateau area at the center is mixed as in FIG. 18) unrelated to partially conventional respiration (continuity of conventional waves) is mixed. In that case, a certain fixed waveform component in such a manner as to depart from an average of phases in comparison therewith, for example, a region different from that in a conventional tendency in a certain fixed range of a waveform element component (shin value) is cut; and a phase (For example, a wave phase whose phase of an inspiration value departs from the range between 90% and 110% of an average during respiration and expiration not reaching upper and lower 50% area at a related amplitude value with the upper and lower wave is eliminated from the calculation. Alternatively, correction is made by being applied thereto in a partially standardizing manner.) at which there is no passage of a certain fixed value, and so forth are calculated. In this manner, a waveform image whose certain fixed quality exhibiting dynamic finding of fixed respiration and blood flow, that is clinically required is ensured can be extracted. In addition, AI may be made to learn a change in value of pixels adjacent to each other, dispersion value, and so forth.

### [Difference in density in an image of an organ]

In the invention according to the present application, a difference in density in an image is displayed by chasing phase shifting of a waveform of an organ. For example, in the case of a lung, a difference in its density is calculated by chasing not the entire lung field but part of phase shifting. That is, a difference in relative density of part of the lung is calculated. In this case, it can be expanded in a fixed area using a Voronoi tessellation method. In the invention according to the present application, densities before and after movement are compared with each other by maintaining the relative positional relationship to output a difference thereof. For example, displaying is carried out in a place of each branch (blood vessels branched from arteries) of blood vessels. Then, a change in density is filtered in a certain place to display a difference thereof. Not the relative position of the entire lung field but an organ (region, constituent element) and organs (a near side of both lobes of a lung, an intermediate lobe, and so forth) that are radially visible are chased.

That is, the movement of each of the blood vessels is chased, and thus filtering processing is carried out for density of each of the blood vessels to display a difference thereof as it is. The movement of a structural body such as an organ or the like can be chased, and a lung shape is formed by being fleshed out from the chasing result. A change in density is displayed a position of each organ, and thus it does not matter whether or not to be the relative positional relationship. When attention is paid to "how to chase a lung field", the point of part of the lung field is chased, and thus an area is extended using Voronoi tessellation. The movement can be chased by determining the shape of the entire lung field, but if a core (point) in a certain block area is determined, the whole shape can be estimated therefrom. It is possible to move the lung field by not performing chasing after determining the frame but chasing the movement of a point after determining the point. In addition, there are some cases where it is not realistic due to a problem of a calculation amount to chase all the points of the organ in an image, and this it is desirable to chase a point by deciding the point in the image of the organ such as a lung or the like. Then, the area of which the point is in charge is determined.

Further, the density may be calculated by eliminating unnecessary frequency with a filter. At a position where the density changes, there are not only a case where the position is shifted, and a case where no position is shifted. That is, this is not the relative positional relationship. In the invention according to the present application, the waveform of an organ can be separated therefrom and grasped by software. It is applicable to take bio-information from not only camera images but also a breastband, an electrocardiogram or the like. Further, the bio-information may often be disturbed by noises. When having the noises from outside, the amplitude suddenly becomes high, and a spike-like waveform appears. This is eliminated. Further, it is also possible to eliminate noises entering on a regular basis. In addition, with regard to how to output images, it is possible to display a change in pixel value in a color image, with a lung shape remaining fixed inside the shape. It is also possible to display the blood flow in the color image by this method.

### [With regard to elimination of specific frequency]

It is possible to eliminate previously known frequencies. Frequencies appearing in an electrocardiograph are eliminated by a filter. For example, a frequency of 50 to 60 Hz, a frequency of 100 to 200 Hz, and a frequency of 0.2 Hz appear to an upper arm, a thigh and an abdomen, and respiration, respectively. Then, the frequencies are eliminated by the filter. In this manner, the frequencies that become noises included in the electrocardiograph can be eliminated, and thus it becomes possible to take out only the element of a heart. In this manner, there are a method of taking out components of heart/heartbeat, and in contrast, a method of eliminating the foregoing. That is, it becomes possible to not only extract necessary frequencies but also eliminate unnecessary frequencies, using the intrinsic vibration number/eigenfrequency originally possessed by an organ. Such a method is applicable to a pulsimeter, a breastband, a camera, an electrocardiogram, and so forth.

FFT processing and band pass filer processing are executed for noise elimination/noise separation. Further, the pulse can be expressed in frequency, and the frequency can be also extracted from the breastband, thereby being extracted/removed from a spectrum. For example, with regard to whether to extract respiration elements included in images of a lung and a heart as respiration, and whether to eliminate them as noises, either is possible.

### [With regard to combination with Al]

AI (Artificial Intelligence) can be used for diagnostic support by making it learn normal images and abnormal images. For example, the thickness of a lung is thin on its outer side, and large in thickness on its inner side. A difference according to respiration in a spirograph is taken, and thus a high or low level is understood. The normal lung has a feature in which "the periphery is thin, and the inner side is thick", and "the slope is small at the beginning of movement, and gradually becomes larger". When visualizing this, color gradually varies with a change therein. Herein, as a slope of how to extend a lung, a volume of the lung is differentiated by time to represent the slope. For example, the portion that looks red is a portion whose density rapidly becomes high, and in the case of the lung, it is shown to be rapidly shrunk. On the other hand, the portion that looks blue is a portion whose density rapidly becomes low it is shown to be rapidly extended. When being mildly abnormal, the lung does not surely move, and thus the portion that does not look red or blue is generated. Further, red and blue are often inverted. According to all or part of the lung, movement of expiration at the time of inspiration, or of inspiration at the time of expiration is seen. Whereas a change thereof remains fixed when being normal, a changing way becomes inverted and becomes uneven. AI is made to learn such the changing way. Then, AI can be made to learn a feature amount according to the changing way. It is possible to perform diagnostic support by making the feature amount correspond to disease/case.

As to preparation of a model obtained via learning by Al, and determination carried out by using the model, in a step of preparing the model by collecting information, the portions where waveforms go up and down are caught by dividing the waveforms for each phase. For example, phases of normal expiration and inspiration are learned. That is, a normal model is first prepared, and an abnormal lung that is not matched with a normal lung is subsequently learned. Next, in the step of being applied to a phenomenon using the model, an output of what percentage covers abnormality is obtained by determination using the model. For example, displayed can be phenomena such that trunks and branches of blood vessels suddenly become thin, disappear, and appear by suddenly increasing density afterwards despite the fact that the blood vessels are thin; phase thereof are shifted; and images are sparsely imaged due to damage thereof. When recognizing these phenomena, diagnosis thereof can be given as being pulmonary thrombosis embolism (PTE). Herein, PTE is exemplified, but this is part of diseases and such phenomena are possible to be generated with respect to other various diseases. For example, the diseases can be grasped by deterioration and increase of signal intensity, variation in reactivity (sensitivity in signal change), signal loss, or the like.

According to the feature of such a PTE patient, as shown in FIGS. 12 and 13, lung bool flow is uneven, and flows in a shifted manner. That is, phase shifting is generated. In the invention according to the present application, a high signal portion is conventionally formed in a radial shape in a morphological manner, and basically seen as a whole when becoming the next phase; and when the phase shifting is generated, it can be determined to be abnormal due to a lesion. It is made possible that AI determines these phenomena. In such a combination with Al, it becomes a feature to chase those moving cyclically.

### [With regard to elimination of artifact]

The artifact with ribs is caught, and needs to be eliminated. In a lung, a structure of branches and leaves of blood vessels is seen at the periphery from pulmonary hilum. This is almost even, and is wholly spread in left/right symmetry. When this is visualized, the branches and leaves of the blood vessels come out, and spaces in between the branches and the leaves are also displayed red. This is displayed by extracting a change in density therefrom as a frequency signal. Herein, the artifact is easy to appear around a diaphragm and a heart. The artifact is also easy to appear on the inner side above the lung. This is because ascending aorta is viewed. AI is made to model movement of the lung for every cycle, and made to learn abnormality to eliminate the artifact.

The present inventors have created a method for eliminating an artifact with ribs. It becomes necessary to distinguish the artifact with ribs from a change in respiration, and the elimination of the artifact is realized by using the following method singly, or using a plurality of the methods in combination. The following method is applicable to any of a perspective image or a difference image of the perspective image, and an image after filter-application. Further, it is possible to identify a rib position as a side effect, and thus not only elimination of ribs but also a situation where the ribs move can be visualized.

The block intensity showing a pixel value of a fine area is calculated for every frame, but in the case of a block where a rib is included in a fine area for only the specific frame, an absolute value of a change in block intensity thereof relatively becomes larger than that of a change in respiration. It becomes possible to specify and eliminate the artifact with ribs by regarding the relatively large change as an artifact of ribs, and making correction. As a correction method, exemplified is the logic that "clamp processing (limiting a numerical value x within the predetermined range [a, b]) is performed by specifying the maximum value and the minimum value", "the artifact is determined by whether or not to exceed the predetermined threshold value", "contraction is made in such a manner that the largest change falls within a threshold value, when exceeding the predetermined threshold value", and "an artifact is taken, when falling outside Weve form as a reference wave by a fixed ratio".

As described above, in an arbitrary block, it is based on an artifact-creating source that a frame, in the fine area of which the rib is included; and a frame, in the fine area of which no rib is included are present. Accordingly, in the arbitrary block, the artifact can be reduced by changing a block shape so as to include ribs in the whole frame, or so as not to include them therein. As a method of changing the block shape, exemplified is the logic that "the block size is made to be large", "a block is made to be a rectangle that is approximately twice as large as a rib size", and "an area of taking an outlier according to a pixel value included in a block is excluded".

There are disadvantages in that resolution is lowered when the block size is made to be larger by the foregoing method. A block in large change in comparison with a peripheral block in a high resolution difference image can be extracted by taking a difference between a low resolution difference image (image obtained by aggregating change in luminance value by making a block size larger) as shown in FIG. 29A and the high resolution difference image (image obtained by aggregating change in luminance value by making the block size smaller) as shown in FIG. 29B. It becomes possible to reduce the artifact while maintaining the resolution, by this method. The difference between the low resolution difference image and the high resolution difference image is divertible as a parameter for characterizing movement of a lung structure, and thus it is suggested that there should be possibility of being able to use this as a parameter exhibiting some clinical significance.

In addition, a method of extracting a high density portion from outside a lung contour to identify it as a bone is conceivable as another artifact elimination method. This is a method of confirming a rib as an artifact by measuring density from outside the lung contour to perform positional calculation. That is, though there are some cases where it becomes problematic to depict ribs, it can be assumed that an original image is a roentgen image, and a high density portion (high density area) indicates the ribs. Then, it appears that the rib portion can be erased by executing masking processing to cancel pixel values each other via superposition. After eliminating ribs, interpolation processing and difference processing are preferably executed.

### [Lung blood flow subjected to learning and determining with Al]

According to a transmission property of a lung in an X-ray image, air enters and lung structure density becomes lower during inspiration, and thus permeability thereof becomes high, and the image looks white. On the other hand, air is reduced during expiration and the lung structure density becomes higher, and thus the permeability becomes low, and the image looks black (dark). Further, as to blood vessels as well, blood of blood vessel lumen is large in quantity and a blood vessel diameter becomes large in a diastole, and thus X-ray permeability becomes low. The blood of blood vessel lumen is small in quantity and the blood vessel diameter becomes small in a systole, and thus the X-ray transmission property becomes high. In this manner, density cyclically varies in images of a lung and blood vessels. According to the present invention, a specific area (an organ or the like) in an image is visualized by tuning such a variation cycle, that is, a frequency.

For example, when visualizing movement of lung blood vessels, the following procedures are carried out.
(1) The frequency of blood flow during imaging is recognized.
(2) A frequency spectrum component table is prepared for difference values of density before and after each area of an XP image.
(3) A frequency obtained by (1) among spectrum components is extracted.
(4) The resulting extracted frequency (respiration frequency) is imaged.

The following feature is observed in a moving image obtained by such procedures. That is, a healthy lung results in a moving image radially expanding from a center. On the other hand, in the case of a patient (for example, PTE: pulmonary thrombosis embolism), blood vessels that should be visible are not observed, thereby resulting in the moving image exhibiting unevenness.

In the present invention, for example, moving images each of 12.5 frames per second are employed. That is, 12 images (pictures) per second can be imaged. Then, the normal lung is examined from two significant viewpoints.

### [Lung blood vessel structure]

According to the normal lung, peripheral blood vessels from main blood vessels look like branches. They are observed so as to flow evenly and radially. The center is visible thick and dark.

### [Flow of capillaries/peripheral lung blood vessels (Normal pulmonary flow)]

An aggregate of fine dots is continuously arranged, thereby looking like a cloud. In this manner, it is because blood flowing speed is high on the pulmonary artery trunk/center side, and the blood flowing speed is low at the peripheral blood vessels that a lung blood vessel structure and capillaries/peripheral lung blood vessels are visible.

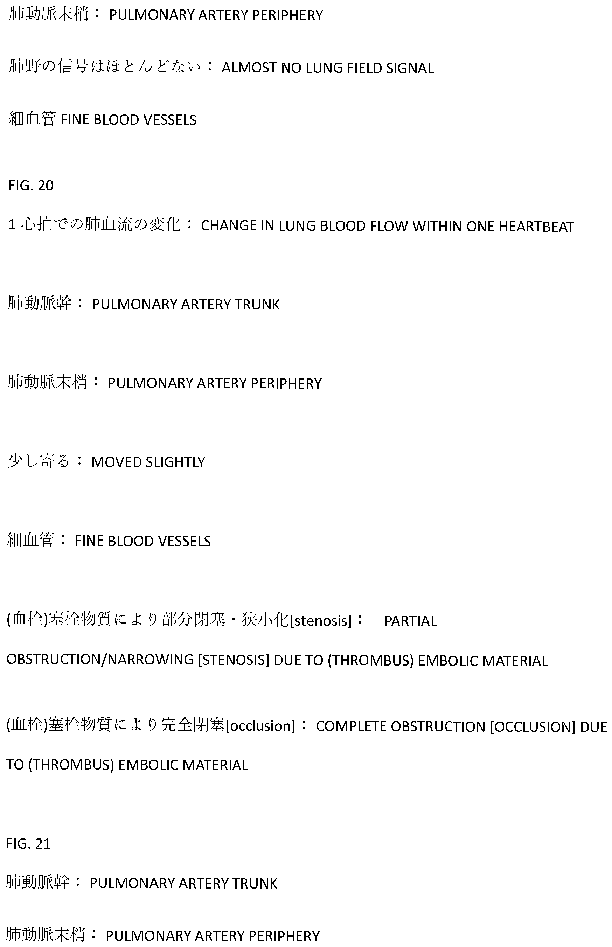

FIG. 19 is a diagram showing a change in lung blood flow within one heartbeat of a normal lung. Herein, shown is one heartbeat for 12 frames. According to a normal lung, respiration is advanced, and blood flow is wholly observed during 45% thereof. The heart takes a rest in the latter half of one heartbeat, and thus blood flow is less observed than in the first half. In contrast, in the case of a patient of pulmonary thrombosis embolism, how to be observed is different therefrom. FIG. 20 is a diagram showing a change in lung blood flow within one heartbeat of an abnormal lung. Herein, shown is one heartbeat for 6 frames. As shown in FIG. 20, the lung blood vessel structure is not observed so as to expand radially from the center, and is uneven. Defective areas are abnormal in the images. The blood vessel structure of the left lung is normal, but the right lung becomes sparse, and thus the blood flow is quite less. The blood vessels themselves are not visible. In the case of a normal lung, the lung blood vessel structure should be clearly and radially visible, but is invisible in FIG. 20. In this manner, it becomes possible to diagnose the lung blood vessel structure. Further, as "MIP image" as well, an abnormal lung has a feature in that slips are present in the image, and thus spots therein are invisible. There are a lot of voids therein, resulting in low density. In this manner, according to the present invention, it is possible to check the blood vessel structure, and it becomes possible to grasp possibility of pulmonary thrombosis embolism based on a display pattern of the image.

### [With regard to flow of capillaries/peripheral lung blood vessels (Normal pulmonary flow)]

In the case of an abnormal lung, no flow of capillaries/peripheral lung blood vessels appears in the image, and thus it can be determined to be abnormal. For example, on the peripheral side of the lung, thickness of the lung becomes thin, but in the case of the abnormal lung, obtained is an image where no part (part looking red in the image) whose density increase appears. In the case of the normal lung, part whose density increases is distributed while showing gradation, but it does not exist in the abnormal case. In this manner, the difference between normality and abnormality is displayed.

When having a suspected case of APE (acute pulmonary embolism), the following feature appears in the image. That is, blood flow appears on the peripheral side of the lung, after respiration action. That is, the delayed blood flow appears in the image. This means that blood starts to flow into closed blood vessels, and thus delayed blood flow appears in the image. That is, in "stenosis: partial closure/narrowing", as shown in FIG. 20, blood flow appears in the latter half of one heartbeat. That is, it becomes visible in the rear of a heartbeat cycle. In "occlusion: complete closure" as shown in FIG. 20, there is no appearance of blood flow in the image. There is no appearance thereof even in the MIP image. In this manner, AI is made to learn images each of an normal lung and an abnormal lung as a subject.

Further, depiction failure abnormality is suspected by thrombus embolus, when no flow of capillaries/peripheral lung blood vessels is depicted (in a phase) immediately after blood vessels of a lung artery. Herein, (a) when the flow of capillaries/peripheral lung blood vessels is depicted with a delay, those in the delayed phase are depicted in a state where blood vessels are thin by "stenosis" or the like; and (b) when no flow of capillaries/peripheral lung blood vessels is depicted in the whole phase, it can be estimated that the blood flow is stopped as "occlusion" by the complete closure.

That is, defected regions in a blood vessel structure can be grasped, and the flow of capillaries/peripheral lung blood vessels of thin blood vessels (terminal vessels), together with the blood vessel structure can be checked. Then, whether or not to suspect "partial closure: stenosis" can be determined by checking a region of not flowing in an early phase (phase immediately after blood vessel depiction), and a region of flowing from a delay phase. Further, whether or not to suspect "complete closure: occlusion" can be determined checking a region of not flowing in the whole phase from the early phase to the delay phase.

For example, there are some cases where blood flow of a right lung rapidly appears in APE. That is, the flow of capillaries/peripheral lung blood vessels often appears in the area that should be closed. This is based on a phenomenon that appears due to rapid blood flow into peripheral blood vessels, because the main stream of blood vessels is stopped, and dammed up.

Further, FIG. 21 is a diagram showing a normal lung, and a lung of PAH {pulmonary arterial hyper tension: pulmonary artery pulmonary hyper tension (including lung arteriosclerosis)}. As to PAH, there is no significant deficiency when wholly viewing the lung, but unevenness thereof appears. The normal blood vessel structure has a configuration of radially extending in the peripheral direction from a center of a lung. Branches are not seen very often. However, when a PAH symptom (abnormality) appears, branches of blood vessels are formed into "irregular shape". That is, when exhibiting abnormality of a lung based on PAH, radial lines in the blood vessel structure are few in number, and rapidly become thinner at the periphery. That is, the blood vessel structure becomes sparse and irregular, and the periphery rapidly becomes thinner. The distribution looks even, but the distribution is at random as viewed in detail, thereby being different from normality thereof. The omission also emerges in the blood vessel structure at the center of a lung. This provides evidence that there is no movement at that portion. Further, the way of blood flow is slow, and appearance of blood vessels becomes slow. For example, the blood vessels are slow by one to two frames, and the blood vessel structure starts to be seen from around a center of the lung. This is abnormal. Further, blood vessels on the peripheral side of the lung are thin, blood flow appears at late timing. This provides evidence that the blood flow is delayed, thereby exhibiting abnormality of a lung. Further, the blood vessel structure itself flickers on the image, and a phenomenon that appears late is observed. Further, cases where the flow of capillaries/peripheral lung blood vessels is not present, is invisible, and is invisible except for a little bit are abnormal. These phenomena are imaged as a difference in comparison with normality.

Herein, the mechanism until a signal of the lung blood vessels is imaged is described. In blood vessels of a normal lung, a pressure difference is first applied to the blood vessels by driving a heart. Next, expansion and contraction are generated by elasticity of the blood vessels. That is, the blood vessels are expanded and contracted by elastic force of the blood vessels. As a result, a change in blood vessel diameter is generated. That is, when being normal, vascular lumen is observed to be large or small in size. Then, the signal is recognized as a change in cyclic XP density, and reflected to an image. Next, when having arteriosclerosis such as PAH or the like, the pressure difference is difficult to be applied to lung blood vessels even if it is generated to drive the heart. Further, expansion and contraction are prevented from being generated by elasticity of blood vessels. This is because expansion and contraction force are lowered due to arteriosclerosis (fibrillation) by lumen thickening. Thus, force by which the pressure difference with a heart is propagated to the peripheral blood vessels is lowered. Further, it is difficult to be reflected to the blood vessel diameter. That is, even when the heart is driven, a large or small change in blood vessel diameter is weaken. As a result, as a cyclic XP density change, those described above are displayed on an image as various abnormal factors. For example, the whole degradation of a signal value (pixel value) is seen. Further, ejection with pulmonary artery is delayed and wholly weak ejection is depicted by preventing the pressure difference from being applied thereto, and preventing expansion and contraction from being generated by elasticity of blood vessels. Further, expansion and contraction are prevented from being generated by elasticity of blood vessels, and thus the flow of capillaries/peripheral lung blood vessels from the center of a lung to the periphery is reduced, and blood vessel phase shadows fall apart. A large or small change in blood vessel diameter is weakened, and thus depiction itself of fine blood vessels from peripheral blood vessels becomes difficult. Then, blood vessels on the center side are club-shaped/branch-shaped, and "diffuse" centering on the periphery is lowered.

From those described above, when elasticity of blood vessels is lost by changing curability (flexibility) of blood vessels, it can be said that it is difficult to depict blood flow thereof. Further, when internal density is difficult to be changed, the signal (pixel) becomes invisible. Appearance of the same image patterns is expected with chronic cardiac failure, hyperparathyroidism, chronic renal failure, connective tissue disease such as amyloidosis or the like, and pulmonary artery curability change as another case other than PAH as described above.

### [With regard to mismatch between ventilation and blood flow]

Conventionally, the mismatch between ventilation and blood flow according to a lung has been diagnosed by lung blood flow scintigraphy. The foregoing relationship is physiologically understood. In the present invention, a situation between the ventilation and the blood flow is simultaneously displayed on an image, and the determination is made by using the respiration and the blood flow in combination. For example, when movement of the lung is almost normal but no lung blood flow responds thereto, it can be determined to be abnormal. For example, when the ventilation is normal but the blood flow is abnormal, there is possibility of lung hypertension, pulmonary thrombosis embolism, or pulmonary emphysema when both of them are poor, thereby resulting in poor blood flow. On the other hand, though the movement of blood flow is normal, there are cases where the movement of a lung is abnormal. For example, the case of unusual respiration with cardiac failure corresponds to the forgoing. When it is hard to breathe though even a physically normal person has one's correct heartbeat corresponds to, for example, the case of choking on food, or the like. Such a model as that of "the mismatch between the ventilation and the blood flow" is produced by mechanical learning with Al.

For example, according to "a disease example of hypoxic pulmonary disease (HLD)", the flow of capillaries/peripheral lung blood vessels is lost. That is, there is a loss area in the lung respiration, and thus the blood flow of the corresponding area also becomes deteriorated. The same phenomenon as that in COPD is observed. In the present invention, diagnosis can be carried out by matching/comparing both the ventilation and the blood flow, and making AI learn them.

### [With regard to correlation with lung scintigraphy]

FIG. 29D is a diagram showing image examples of MBDP (Maximum Blood-flow-related Differential Projection) and ABDP (Accumulated Blood-flow-related Differential Projection). Whereas MBDP means "for each block, the largest value is selected between frames, and is projected on one image", ABDP means that "for each block, a value obtained by adding a change value in a plus direction thereto is projected on one image". Further, the method of utilizing MBDP and ABDP aims at obtaining a clinical index by dividing an image of a lung image into 6 areas to calculate average/distribution of intensity values in respective areas, as shown in FIG. 29E.

For example, the correlation with a lung scintigraphy (evaluation method of existing lung blood flow) in between can be taken. According to the lung scintigraphy, a radioactive medicine is injected; "an accumulation degree of blood" is measured by acquiring "counts of a radioactive substance"; and it is a feature that a signal of a region where the blood remains stuffed becomes high. In the lung scintigraphy, the lung image is similarly divided into 6 areas to acquire counts of a radioactive substance in each area. In the lung scintigraphy, evaluations are relatively made, and thus, for example, when the counts are made to be "100, 200, 300, 400, 500, and 600", "4.7%, 9.5%, 14.3%, 19%, 23.9%, and 28.6%" are calculated as a ratio to a total count of 2100. In MBDP and ABDP as well, the total intensity of each area is replaced with counts of the lung scintigraphy, thereby being able to be evaluated as a ratio to the total intensity. Thus, it becomes possible to obtain an image close to the scintigraphy. For example, even when counting respiration and blood flow, whether or not respiration and blood flow are slow or fast, or it is substantially flowing is counted as a signal. The total amount can be grasped as a flow, thereby contributing to a diagnostic support.

### [With regard to events each having reproducibility]

In the present invention, AI is made to mechanically learn events repetitively appearing many times, that is, events each having reproducibility. Thus, it is possible to employ a method of determining abnormal by making it learn normal states, and a method of outputting disease candidates by making it learn abnormal states. A method of outputting a feature amount of each disease example is employed as an outputting method of AI determination. This feature amount can be determined based on an image pattern (an average pixel value in each area, a rate of change in pixel value, or the like), and includes a plurality of diseases.

### [With regard to lung blood flow and Al]

FIG. 30A is a diagram showing lung blood flow during one heartbeat of a heart for every frame. FIG. 30A shows a situation of lung blood flow during one heartbeat with 10 frames. The flow of one heartbeat of lung blood flow starts to flow from blood vessels on a center side of a lung, and subsequently expands to peripheral terminal vessels. The terminal vessels each have a small diameter (small blood vessel lumen) and slow flowing speed, together with those that are present in large number; and thus the image looks like a cloud expanding with a delay after the center. That is, after the main (Main pulmonary artery, distal pulmonary artery) blood flow is seen, terminal vessels become visible.

FIG. 30B is a diagram showing measurement result examples of MPA (Main Pulmonary Artery), DPA (Distal Pulmonary Artery), and DPV (Distal Pulmonary Vein). DPA is observed with a slight delay after MPA, but a rapid decline occurs from MPA. Further, the above-described TV (Terminal Vessels) appears before DPV. In FIG. 30A, as in the measurement result shown in FIG. 30B, after blood vessels are visible in the first 2 or 3 frames toward the periphery from the center, fine blood vessels appear from after 4, 5 or 6 frames.

As to how AI is made to learn this, "bullseye" for concentrically displaying a lung and a heart is used. The blood vessels expand from pulmonary hilum in a substantially concentrical manner, but there is a phase at which the pixel value of the blood vessels is made to be a peak (the third or fourth frame in FIG. 30A). Herein, both a blood vessel structure and peripheral blood vessels are included. Then, the peak of the pixel value is first detected to grasp the signal (pixel value) of lung blood flow. Then, there is a phase at which the blood vessel structure is visible on the near side of the peak, and thus this is extracted. According to this detection method, there are (1) a method by which "a blood vessel structure is extracted"; and (2) a method by which "lung blood flow flows from pulmonary hilum to the periphery, and thus extraction thereof is made at the pulmonary hilum or on its near side.

For example, according to the lung of a patient of acute pulmonary embolism (APE), the blood vessel structure become coarse, and thus as to the concentric circle of the bullseye, the lung field area divided into approximately 17 equal parts is considered. When concentrically distributing a lung shape, what happens during a phase at which the blood flow flows, and another phase of the blood flow of terminal vessels can be seen. Then, making into AI is facilitated by displaying variations of the distribution. This is because normality and abnormality are easily distinguished from each other by the variations of the distribution. That is, the pixel values in the area where the blood flow is present, and the area where no blood flow is present are digitized via division thereof by every area in an airspace manner. According to this, automatic calculation is possible. Digitization can be made available, and thus AI also becomes applicable. The calculation can be made according to segmentations by this method, and loss portions and so forth can be detected.

With regard to terminal vessels, when subtracting a pixel value in the area of a blood vessel structure from the whole, the pixel value of the terminal vessels can be obtained. That is, the remaining portion becomes the terminal vessels by excluding the blood vessel structure, and thus what kind of blood flow the terminal vessels become can be shown.

Further, in a stereoscopic structure, which part is lost (whether or not the blood flow exists), or the like can be detected. The case of 3D or 4D is also applied similarly, and displaying can be made possible. That is, the near side of a portion where the pixel value (signal) of the blood vessel structure is high is detected to determine a phase thereat by dividing the blood vessel structure into areas. By this method, the blood vessel structure can be detected; the terminal vessels are also detected; and how the blood flow (flow) stands can be displayed. When collecting these signals, such a bar graph as to go down after going up is shown, and thus what kind of movement is done in blood vessels inside the area can be displayed. Further, what kind of movement is done in terminal vessels inside the area can be displayed. How those described above flow as a whole can also be displayed. When these are calculated in one heartbeat, waveforms as shown in FIG. 30B are obtained.

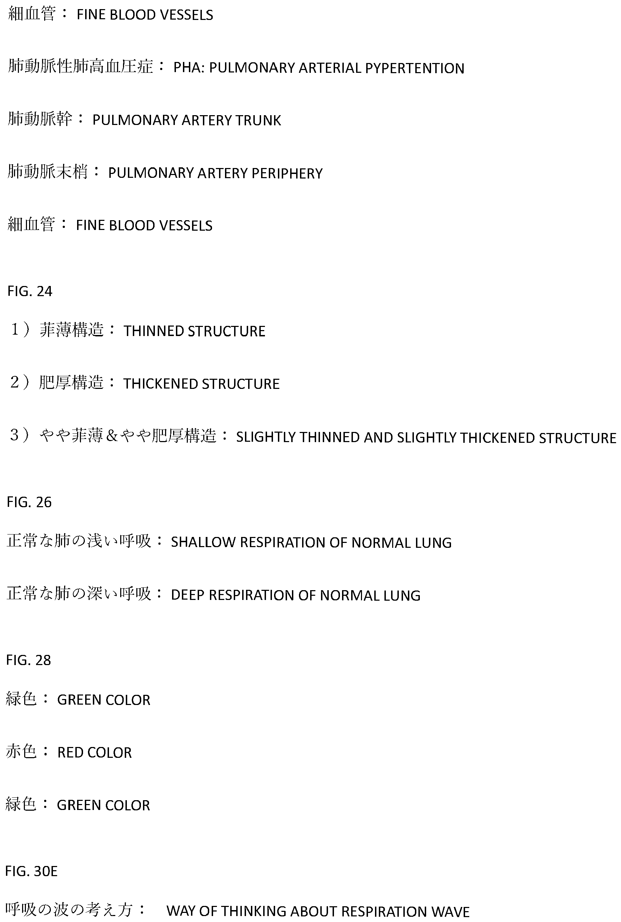

FIG. 30C and FIG. 30D each are a diagram showing signal intensity according to an ultrasonic image of an atrium. In FIG. 30C, E representing a peak signal and A representing a signal that indicates influence of the movement of the atrium are observed. In FIG. 30D, an aftereffect signal L appears between the signal E and the signal A representing a signal that indicates influence of the movement of the atrium. When L appears between E representing the peak signal and A representing the signal, possibility of cardiac failure is considered. In this manner, lesion is possible to be predicted via graphing and digitization by grasping the difference with respect to the normal lung.

Further, a comparison method is known by superposing past and present waveforms by an electrocardiogram, but comparison between before and after treatment is made; a healthy normal person is compared with a patient; and comparison between the same patients in the past is made by superposing waves shown in red, blue and green, for example, and automatic diagnosis is made possible. For example, the automatic diagnosis is made possible by grasping such a manner that a T wave is shifted, and rising after a peak is unstable, and medical doctors can even write the observations.

### [Learning and determination done by AI with respect to respiration of lung]

As to respiration the lung, tunability of change inside the lung field is chased. That is, whether or not a situation where lung movement expands from the center of the lung to the end-stage is correct is determined. Specifically, the linkage of the movements of diaphragm, ribs, and lung tissues is observed. In addition, greater pectoral muscle may be added therein. Further, as to an image, a degree of gap can be evaluated. Even if being a normal lung, there are some cases where a viewing way is different therefrom according to natural respiration or effort-respiration, but these are determined as normal lungs. Further, the determination can be made with "linkage", based on "area to which change in lung movement is propagated", "continuity of blood flow, flowing situation", "continuity of movement from the center of the lung to the end-stage", and "movement itself of the diaphragm".

More specifically, with one wave (for example, wave of respiration element) extracted from the image as a reference, the evaluation is made with the linkage with respect to it. The movement of the lung is centralized to the wave; a large or small amplitude, phase shifting, and a vector direction are checked; and AI is made to determine them, in addition to anatomical knowledge. In this case, a determination target is to be changed depending on which wave is extracted from the image. That is, a frequency specific to disease (disease example) and phase shifting are present, and thus the disease (disease example) is identified based on detected frequency and phase shifting. The portion to which the lung is tightly fixed due to tuberculosis becomes a frequency of 0. For example, in the case of myocardiopathy among disease example each whose neurotransmitter is difficult, a WEB form is delayed together with phase shifting, and the waveform is different therefrom. The frequency specific to the disease is present, and thus a frequency tunability image specific to the disease can be output. Thus, it becomes possible that a new feature amount is obtained, and a new image is output.

In the present invention, images are formed according to a respiration frequency. That is, when an image according to the respiration frequency is extracted, an image of lung movement caused by respiration becomes visible. Herein, a differential value of the lung movement is imaged. Such an image only extracts a change along the respiration frequency in an X-ray image. A change in density is chased, and thus it is because the density is highly enhanced (green, yellow, red) to become white according to XP (lowered permeability); it is because the density is lowered (green, light blue, blue) to become dark according to XP (improved permeability); and such a change is visualized by images. In addition, the image according to the present invention is reversed to a graph of inspiration/expiration via thorough pulmonary function examination.

As to a signal change in lung movement, a signal value becomes higher toward trachea/bronchial (center), narrow bronchial, and alveolus (periphery) at the beginning of respiration; and a signal value becomes lower toward alveolus (periphery), narrow bronchial, and trachea/bronchial (center) at the end of respiration. This is applied to both inspiration and expiration. XP is similarly applied thereto, and in the case of inspiration, the case where permeability becomes high means that density becomes low, and the difference becomes a minus vector (color exhibiting blue). On the other hand. in the case of expiration, the case where permeability becomes low means that density becomes high, and the difference becomes a plus vector (color exhibiting red).

FIG. 22 is a diagram showing an image of a normal lung. The respiration causes a change from light blue to blue (density vector strengthened to negative in value) while motion in the inspiration direction (permeability becoming high) is strengthened. On the other hand, the respiration causes a change from yellow to red (density vector strengthened to positive in value) while motion in the expiration direction (permeability becoming low) is strengthened. As the lung is on a thinner outer side, the signal becomes weaker as a whole, but as the lung is on a thicker inner side or diaphragm side, the signal becomes higher as a whole. The signal becomes larger or smaller in intensity according to waves in a moving way of respiration "inspiration, expiration".

### [With regard to responding to image and disease]

FIG. 23 is a diagram displaying a normal lung and a lung of COPD. In the case of a normal lung, a form of expanding to "bronchi ^{~} segmental bronchi ^{~} fine bronchi ^{~} alveolus is seen, but in the case of the lung of COPD, the form of expanding only to "bronchi ^{~} segmental bronchi" is seen, and thus no expansion to "fine bronchi ^{~} alveolus terminal" is grasped. In this manner, in the case of the lung of COPD, an uneven speckled image is visible at the central portion of the lung. Further, left/right asymmetry also results in determination to be abnormal. The linkage is also applied thereto. The diaphragm, ribs, and lung tissues result in movement thereof in a separate manner. In the present invention, the linkage between a wave as a reference and each structure is evaluated in association with an anatomical structure.

FIG. 24 is a diagram showing a stereoscopic structure on pulmonary apex-diaphragm side. As shown in FIG. 24, according to "pulmonary apex", the lung structure is stereoscopically thin. Thus, a change itself becomes small in the image according to the present invention. Further, extraction failures caused by superposition during lesion are suppressed. The lung structure is stereoscopically thick "on the diaphragm". Thus, the change largely appears in images according to the present invention. Further, extraction failures caused by superposition during lesion become large. In the images according to the present invention, it can be said that the change is a little large "slightly on the tail side of pulmonary apex (below pulmonary apex)". The superposition during lesion also becomes moderate. For example, a dependence difference is easy to appear by a degree of thickness, depending on the lung structure. The soft part shadow is strong "below the diaphragm", and thus the difference of each XP image is difficult to be numerically expressed, thereby not being easy to be evaluated.

Therefore, even if to be a normal lung, there are some cases where the following events appear.
- Decline in change rate of peripheral lung field
- Decline of a lung structure around interlobar fissure
- Unevenness of a change due to movement of a structure near pulmonary marking
- Mild decline in signal inside a lower right lung field
- Change in thickness before and after thorax, and in thickness of a lung on the side of pulmonary apex such as greater pectoral muscle or the like.
- Artifact of bones of ribs
- Shadow of only plasticine branches

FIG. 25 is a diagram showing a situation of peripheral lung field. A lobular structure is stereoscopic and complicated in the peripheral lung field, and this is reflected to the image, thereby resulting in plasticine peripheral change. There are some cases where an expanding way of the peripheral lung field is relatively lowered via a lung shape, extraction of ribs, extraction of greater pectoral muscle or the like. With respect to a viewing way of the side edge of the lung, there are some cases where differences are produced in extraction ability of alveolus from peripheral fine bronchi. This varies depending on a respiration degree and a respiration situation. That is, a signal change according to a respiration cycle appears uniformly. Further, a change to be thin or thick from a pulmonary apex part to the diaphragm side, or from an outer side of a lung to an inner side thereof appears according to the respiration cycle. However, this is generally in left/right symmetry. In the present invention, whether or not lung movement and movement of waves as a respiration cycle are linked with each other is evaluated. Whether or not thorax, diaphragm, ribs and so forth move in linkage with waves as a respiration cycle is evaluated.

As to a lung structure around interlobar fissure, a decline in signal value (pixel value) is observed. At the side edge of interlobar fissure of a lower left lung field, a change in alveolus density is specifically lowered, and a signal change as well hardly appears. Further, a mild decline in signal inside a lower right lung field is seen, but corrections are made in order to make this be within a normal range, though as to this, there are cases where a negative signal is observed from a structure with below the diaphragm. In the present invention, when determining a normal moving image, images are read while carrying out comprehensive observations, by using a stereoscopic chest structure and a respiratory physiological change rate in combination. In addition to those described above, various things such as a partial function of a lung, or the like can be determined by reading images while taking into consideration various structures according to XP, and image conversion thereof.

When performing mechanical learning and determining with Al, waves as a reference of a respiration cycle or the like are defined, and whether or not to be in linkage with the waves as a reference thereof becomes a determination point. That is, this is linked with waves, determination is made from a viewpoint of whether to be a large variation, a small variation, or no variation with respect to the waves. Herein, when one whose variation should be large is small, and one whose variation should be small is large is determined to be abnormal. Further, when the phase is shifted to waves as a reference is also determined to be abnormal. That is, one exhibiting a fixed linkage with waves formed from a respiration cycle is regarded as a normal lung. A normal lung model is obtained by making AI learn this. According to the AI determination, whether the linkage is held or lost is determined. Further, there are cases where the amplitude is abnormal, and the phase is abnormal.

FIG. 26 is a diagram showing shallow respiration and deep respiration of a normal lung. In the case of the shallow respiration of the normal lung, a wave slope in change is weak, undergoing less change, and thus periphery (alveolus from peripheral fine bronchi) cannot be depicted. In this case, only fine bronchi from bronchi is depicted. On the other hand, in the case of the deep respiration of the normal lung, a wave slope in change is large, undergoing sufficiently large change, and thus periphery (alveolus from peripheral fine bronchi) can be depicted. In addition, in the case of deep respiration of an abnormal lung, movement thereof is delayed with respect to movement of the diaphragm. In this manner, in the present invention, the respiration is evaluated by "linkage", and thus diagnosis is made possible by the linkage.

FIG. 30E is a diagram showing movement of a respiration wave. When a waveform slope is large, when being displayed on an image, those displayed thereon are clearly visible. This is because everything is linked with the waveform and the slope. In the case of a normal lung, the diaphragm, thorax, trachea, and an output way of a signal become similar thereto. The diagnosis is made possible by graphing how much they match with the waves.

Further, how thick or thin those before and after the lung are can be grasped as a stereoscopical structure. For example, according to a chest XP image, CT is displayed by slice images, but stereoscopy is shown via superposition. FIG. 30F is a diagram showing a stereoscopic structure and a cross-section by chest XP images. The outer side of the lung is stereoscopically thin, and an image change is reduced. When there is lesion, a malfunction degree in image depiction via superposition is lowered. The inner side of the lung is stereoscopically thick, and an image change becomes large. When there is lesion, a malfunction degree in image depiction via superposition becomes large. On the slightly inner side (intermediate layer outer side) from the outer side, an image change is large to a certain extent, and when there is lesion, a malfunction degree in image depiction via superposition becomes large to a certain extent.

FIG. 24 is a diagram showing the side face of the lung. Similarly, as viewed from the side, the thickness in front and rear thereof is related thereto. It can be grasped that the pixel value is increased or not increased in a stereoscopic structure. Whether or not to be viewed to be expanded for every structure or stereoscopic structure of the lung can be displayed. In the case of shallow respiration, expansion of the lung is small, and thus a signal is not output largely. If the wave is based on a source, displaying is made possible by grasping a slope, thereby being usable for the diagnosis. With regard to a tracheal structure itself as well, viewing should be easy at the beginning of expansion (because of a large slope in change), but something become wrong herewith when not being visible, and thus possibility of having lesion can be predicted. Further, it can be determined that something is wrong therewith when not being visible at a time to be visible on the center side of the trachea. The amplitude moves along the stereoscopic structure and waveform. When not moving along them, it can be determined to be abnormal. As to whether or not to take the same amplitude, when it is not identical thereto can be determined to be abnormal.

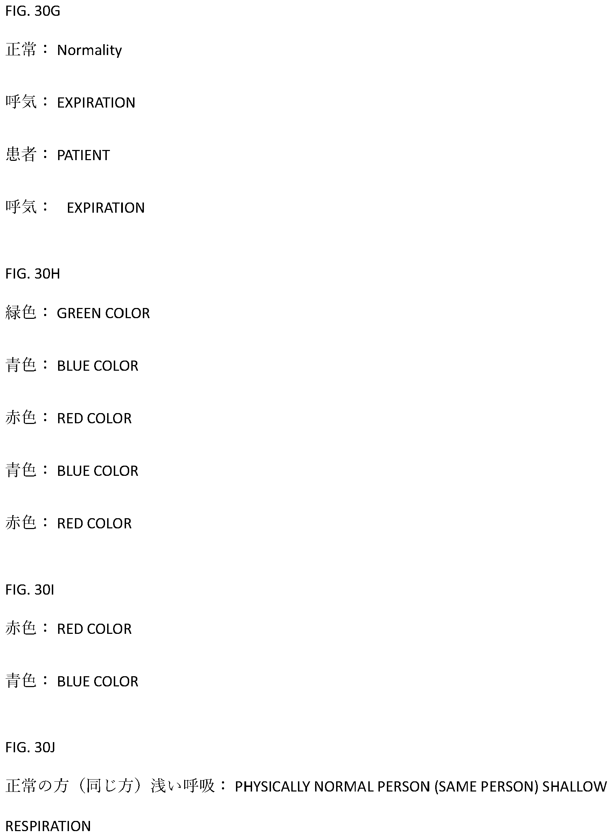

FIG. 30G is a diagram showing a normal lung and a patient's lung. FIG. 30H is a diagram showing a patient's lung. The "red color" portion among images of these lungs exhibits an enhanced pixel value, and indicates an expiring state out of respiration. The area of a lung as well as the volume is reduced, and thus the pixel value becomes plus. The "blue color" portion exhibits a low pixel value, and thus indicates an inspiring state out of respiration. The area of a lung as well as the volume is increased, and thus the pixel value becomes minus. These are displayed to be abnormal in the movement. According to a patient's lung, blue color is displayed at the time of red color to be observed, and red color is displayed at the time of blue color to be observed. That is, the movement opposite to the original one is performed, and thus it can be determined to be abnormal. Even when no color change appears despite that fact that the diaphragm is in motion, it can be determined to be abnormal. For example, after AI is made to learn an image of movement of a normal lung to construct a learning model, existence of lesion can be determined with respect to the image of a patient's lung. Further, an image feature of a normal lung is digitized, and it can be determined to be abnormal, in comparison to one obtained by digitizing a feature of an image of a patient's lung.

FIG. 30I is a diagram showing a patient's lung. In this case, a blood vessel structure at the center of the lung is visible, but a peripheral blood vessel structure is invisible. No peripheral alveolus is in motion. A normal lung has the wave correlation, but no abnormal lung has the wave correlation. With regard to this, AI can execute learning and determination. Further, digitizing is carried out within a fixed area, and how much different they are from each other can also be grasped. FIG. 30J is a diagram showing comparison between shallow respiration and deep respiration. When the respiration is shallow, the image is light; and when the respiration is deep, the image is deep. Segmentation is calculated in accordance with the waveform to make a graph.

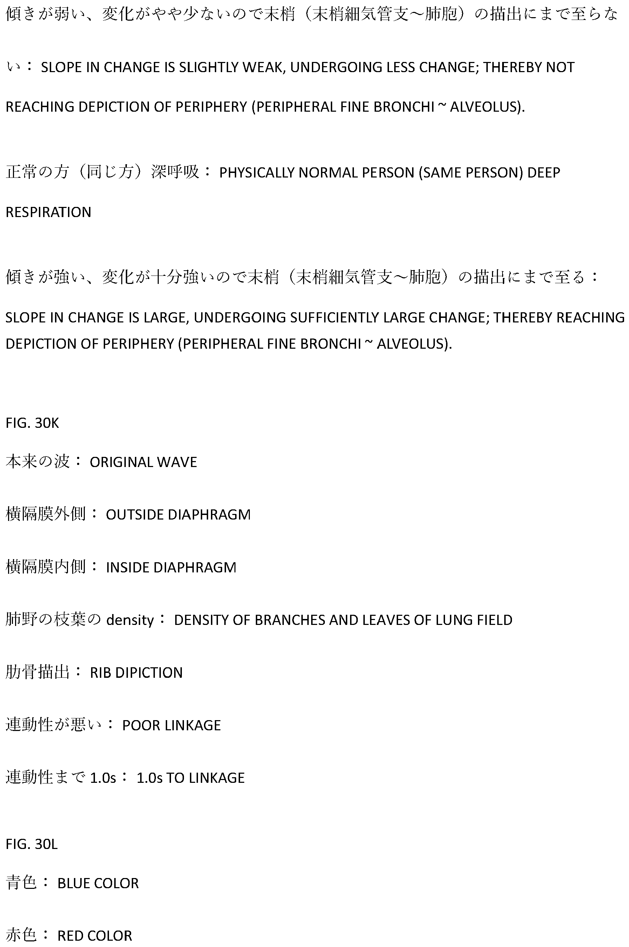

FIG. 30K is a diagram showing a state where waves of diaphragm or the like are shifted with respect to the original wave. The shifting is based on phase shifting. For example, in the case of a lung of an interstitial pneumonia patient, as shown in FIG. 30K, the phase is shifted with respect to the original wave. The phase shifting is digitized. One that is effectively applicable is specifically a heart. When the neurotransmitter is delayed, a myocardium moves with a delay. The movement of the myocardium with a delay means that the phase is delayed. The foregoing phase shifting is digitized and pixelated. When a transmission delay of (disorder of) the neurotransmitter is observed, it is likely to develop myocardial infarction after one or two years. That is, whether or not the amplitude of waves is shifted, and whether or not the phase of waves is shifted are digitized or pixelated.

In the case of COPD, the phase of pixel change of the lung is shifted. The movement is shifted with respect to a pulmonary apex area, response thereto is poor. According to an interstitial pneumonia patient as well, the phase in pixel value change of the lung is shifted. In contrast, according to a healthy normal person, no phase is shifted. The determination becomes possible by learning such a phase shifting pattern with Al. Specifically, it is difficult to be grasped with the naked eye, and thus it is useful to be digitized, and to make AI learn and determine it.

FIG. 30L is a diagram showing an image of a pneumonia patient. It is displayed that a lung structure is lost. It is normal that a blood vessel structure radially extends from pulmonary hilum, but is fully displayed at random. This can be determined to be abnormal. Further, according to a patient partially having fluid retention in the chest, as shown in FIG. 30L, the blood vessel structure is also displayed at random. In such a case, white spots also appear in the roentgen image, and thus diagnosis can be carried out together with the foregoing by a medical doctor. That is, it is possible to be collectively determined by using a roentgen image in addition to an image of only the respiration, an image of only the blood flow, and an image obtained by using these images in combination.

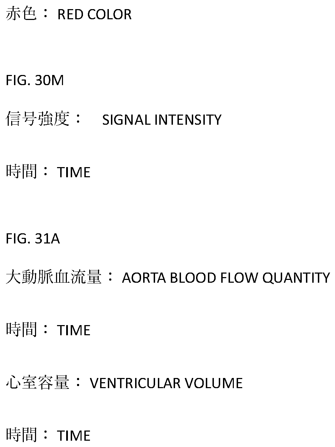

FIG. 30M is a diagram showing one example of electrocardiogram. As shown in FIG. 30M, a signal whose cycle is shifted other than a pulse portion shown by a fixed cycle is often displayed. This is the main reason why a muscle movement, a lung movement, and a creak of ribs have been put in electrocardiogram data as noises. That is, shifting generated in the electrocardiogram data results by receiving influence of another organ or region. Conventionally, the shifting is manually corrected, but in the invention according to the present application, this correction is processed automatically. That is, processing is done as frequency tunability data by setting the foregoing data to "frequency tunable data". More specifically, only the frequency of a cyclic pulse signal that appears in the electrocardiogram is taken via filtering by Fourier-transforming the data, and the electrocardiogram data from which noises are removed can be obtained after finally performing inverse Fourier transform therefor. Noises of respiration can also be removed therefrom in a similar manner.

### [With regard to lung of cardiac failure patient]

Further, FIGS. 27 and 28 each are a diagram showing a lung of a cardiac failure patient. FIG. 27 shows one embodiment at the early stage out of the former half of one heartbeat, and FIG. 28 shows one embodiment at the latter stage out of the former half of the one heartbeat. As to a normal blood vessel structure, a configuration of radially extending in a peripheral direction from the center of a lung is employed, and branches are generally invisible. However, according to the case of a cardiac failure patient, the branches of lung blood vessels each become "distortion-shaped". That is, in the case of lung abnormality existing based on cardiac failure, the blood vessel structure has few radial lines in number, and becomes rapidly thinner at the periphery. That is, the blood vessels each are sparse, distorted, and become extremely thin at the periphery. Though evenly being viewed as a distribution, when viewed in detail, the blood vessels become "coarse", and it can be said that they are "diffuse-coarse". This is different from normality. The flow of capillaries/peripheral lung blood vessels can be observed, but the delay of blood flow appears as a whole. The movement is wholly shifted, and thus a phase delay is generated as a blood flow wave. Further, as shown in FIG. 28, even in the time zone where the blood flow is completed, the blood flow is observed. This indicates that the blood flow appears from pulmonary hilum with a delay. In the case of a normal lung, the blood flow is observed in the time zone where no blood flow is observed. These phenomena are visualized as being different from normality.

### [With regard to 3D tomography]

According to a three-dimensional image display technique, a projection image based on three-dimensional information is displayed onto an image display device such as LCD as being a two-dimensional plane, or the like. The three-dimensional image display technique is classified into a method of projection onto a two-dimensional plane as an image display face (parallel projection, perspective projection), and an image display method (surface/rendering, volume/rendering, maximum value display, minimum value display, sum value projection, and so forth). Further, it is understood that a multi-slice construction method (MPR) that makes an observer be in association with three-dimensions by performing arbitrary cross-sectional displaying without using the projection method is also included in a three-dimensional display system.

It is conventionally carried out that a subject is conveyed into a CT room during IVR (interventional Radiology) to image tomographic images, and an IVR-CT device is used. Herein, a C arm CT imaging technique with a blood vessel imaging device provided with a C arm avoids a conveyance risk by instantly obtaining tomographic images during IVR, and further realizes excellent cost performance in terms of operation and cost in comparison to the IVR-CT device. There is provided a 3D ANGIO technique as a technique of mainly reconstructing tomographic images from rotation images by the C arm, but whereas imaged blood vessels are mainly depicted by the 3D ANGIO technique, the C arm CT imaging exhibits significantly excellent low contrast resolution, and makes it possible to depict light tumor deep dyeing, or the like.

In image processing according to the C arm CT imaging, as to the imaged rotation image, tomographic images can be obtained via three-stage image processing comprising conversion processing into water permeability length, reconstruction pre-processing, and reconstruction processing. In the conversion processing into water permeability length at the initial stage, the pixel value of the rotation image is converted into the water permeability length. The water permeability length is an amount obtained by converting a certain pixel value into one in which it corresponds to how much millimeter water is penetrated, when an X-ray condition is identical thereto. Specifically, after gain correction of a detector, a scattered radiation component is corrected by scattered radiation correction processing, and converted into the water permeability length during beam hardening correction processing. The dark portion in the rotation image means that X-ray absorption is large, and thus the converted water permeability length becomes longer, and the image after conversion thereinto becomes negatively/positively inverted.

For the following reconstruction pre-processing carried out, examples of four kinds of correction processing include C arm orbit correction, ring artifact correction, truncation correction, and π correction. The C arm orbit correction means that shifting between a real C arm orbit and a theoretically idealistic orbit is corrected. The ring artifact correction is a correction for reducing a ring-shaped artifact in a tomographic image, that interrupts image observation. The truncation correction is a correction for improving flatness of a tomographic image by interpolating sinogram thereof for a subject portion protruding from a visual field during rotation. The π correction is one for correcting the difference in correction data amount generated between the central portion and the peripheral portion, when performing half scan reconstruction for the cone beam effect. After correcting those described above, the tomographic image is calculated by the reconstruction processing. Algorithm of the reconstruction processing is referred to as a FBP (Filtered Back Projection) method.

In the C arm CT imaging, imaging is made from various angles, taking time of about 30 minutes. For example, the cycle of respiration is not fixed, and thus the correction is made, using reconstruction. Herein, the region near a tube lamp is clearly visible, but the region at a distance is not clearly visible. It becomes visible when a voltage is raised, but processing of eliminating noises need to be carried out by taking the difference while the permeabilities remain unchanged. For example, not only the front but also the back becomes visible by subtracting one when viewing only a near region from another when viewing the region from behind. Though being wholly visible when the voltage is raised, things become complicated, and thus the deep side is made easily visible by subtracting the near side therefrom, and in contrast, the near side is made easily visible by subtracting the deep side therefrom.

No method of making visible while correcting an image in a respiration state has been realized in the past. In the invention according to the present application, inspiration action and expiration action are fixedly matched with each other. Reconstruction is carried out by matching a frequency and a phase therewith. That is, under a plurality of conditions, processing is mutually carried out to prepare a tomographic image by changing the plurality of conditions, using a plurality of images imaged from various directions. Then, this is used in roentgen imaging. That is, the tomographic image in the roentgen image obtained via frequency analysis is prepared. Fine adjustment is finally carried out via reconstruction. As to specific organs, tomographic images are prepared at a plurality of places under a plurality of conditions. For example, as to a lung and blood vessels in motion, waves are matched therewith with a phase as a reference. As to respiration as well, phase matching is carried out. Then, positioning and stereoscopical feeling are produced during imaging. Blood vessels as well need to be matched therewith in time (synchronization). In this case, for example, after being imaged in 10 heartbeats, the phase is matched therewith.

In the invention according to the present application, imaging is made while being in motion, and thus the frequency of an organ is taken into consideration. Accordingly, those such as temporal aspect, positioning of phases, phase matching, and further angle adjustment are performed. In the case of the C arm CT imaging, the imaging place with respect to the organ is not completely identical thereto, and thus positioning and angles matched with each other become necessary. For example, when imaging a lung at an angle of 45 degrees from diagonally forward right, there are some cases where it actually becomes an angle of 43 degrees, and becomes an angle of 49 degrees. Such shifting needs to be finely adjusted based on the positional relationship between ribs and thorax.

That is, positioning is carried out so as to match with positions and angles of "centrum, heart/vertical kuo structure, ribs, and so forth" acquired beforehand from CT images, C arm CT tomographic images and XP images additionally acquired from various angles, and additionally with 3D images of bronchi and a structure such as the bronchi. The high concordance exhibits "centrum, heart/vertical kuo structure, and ribs" in this order, but a cross-section and a stereoscopic 3D structure are prepared based on information during preparation of a stereoscopic structure to perform 4D preparation by specifically grasping the most matching angle and positional relationship among imaged XP images as image patterns, using AI or the like, while strengthening a coefficient of matching angles as well as positions of the above-described centrum, and slightly lowering a matching coefficient via lung movement such as ribs. Specifically, the CT image is an "inspiration image" imaged during subject's inspiration, and thus it appears that most matching easily results in the origin of an XP image during the maximum effort inspiration. Further, when imaging XP from multidimensional directions, there are some cases where such angle imaging is calculated from a standard image via information about age and height, when not previously having images each specific to a subject, such as CT, and C arm CT tomographic images to prepare a cross-sectional structure and a stereoscopic structure obtained via fine adjustment in quantity, and further a stereoscopic cross-sectional structure and a stereoscopic structure according to movement of respiration and an organ such as a heart or the like.

A fine pulmonary thrombosis state is intentionally prepared by injecting albumin into a patient. In the case of a normal lung, albumin is distributed in the whole lung. When suffering from pulmonary thrombosis embolism, the end-stage is clogged, and thus no albumin reaches it. Therefore, unevenness is generated in the image. When being viewed by an integrated structure of 3D, it is understood that the front side (front side of the lung) is put in, but the rear side (back side of the lung) is not put in. Then, a tomographic figure is prepared from 3D. When phase shifting is visually large, not readily flowing is seen, and thus it is understood to be abnormal.

Further, In the case of roentgen imaging of AP (anterior-posterior), (1) there visibly appears in a near region from a tube lump, that is, a lung, or on a front face (A) side possibly due to a distance thereof from the detector. (2) Since imaging is made many times, imaging is also made in a state where the dose hardly reaches the deep side (P side) in the environment whose dose is insufficient (low dose). Thus, according to transparently visible confirmation default imaging, it is stronger (on the A side in AP imaging, and on the P side in PA imaging) in a state close to the tube lump side; and in the region close to the detector {distant from the tube lump (on the P side in AP imaging, and on the A side in PA imaging)}, relative change and a signal thereof can be identified less than on the tube lump side. Thus, (1) the front side imaging, the whole side imaging, and the rear side imaging are separately provided by applying the same imaging to a high dose region (even image quality thereby, or an image further emphasizing imaging on the P side), and performing conventional low dose imaging. (2) It is made possible to image faces by AP imaging, and PA imaging, respectively. (3) The signal is often delayed on the lower lobe back side, and a signal difference is easy to be generated due to the phase. (4) Under respiration, the more rearward side image is easy to be depicted with respect to breath-holding. (5) The deep respiration in comparison to shallow respiration and normal respiration is easy to exhibit a significant change.

By using the above-described tendency, superposing both of them (the whole signal ratio objectively depicted by superposing AP and PA), and performing subtraction between them after including the foregoing; imaging is carried out by emphasizing the front side (A side) or the deep side (P side) according to "whether the front side is prioritized or the whole is set to plus", "whether the rear side is slightly prioritized or not", and "those more clearly observed high in voltage (high voltage > low voltage)". Further, imaging from multidimensional directions is carried out after including RAO and LAO, an RL direction, and so forth. Further, imaging is performed on the tube lump side multidimensionally in various ways by carrying out the imaging while being rotated, and spirally rotated. Respiration is not wholly fixed, and thus signals are matched with each other at the position and relative phase by superposing the standard lung and the patient's standard one on a tomographic 3D image simultaneously imaged, using dilation, contraction, and temporary reconstruction standardized with a master waveform or the like as a base, and rough respiration on the cross-section, and visualization of blood elements are enabled. Further, a multidimensional 4D image of time applied to three dimensions, and a signal on each cross-section can be prepared via multidimensional permeability imaging.

### [With regard to a method of applying pulses to a human body]

ASL (Arterial spin labeling: Arterial spin labeling method) is an imaging method of acquiring perfusion images and relative cerebral blood flow amount images without using any imaging contrast agency, by labeling arterial blood flowing onto imaging cross-section, using RF pulses. The pulses that are an impact signal become marking when periodically applying it thereto, and how far a neck is rotated is understood. For example, when the RF pulses are applied to the neck, the pulses are diffused over the whole head. It is normal that the pulses are diffused thereto, but there are no diffusion cases. Herein, the frequency tunability can also be recognized in the head and estimated, and it is estimated that whether or not the blood flow is easy to flow can be grasped. The frequency applied to a human body is clear, and thus whether to be normal or abnormal can be determined by being shifted or delayed from this frequency.

### [With regard to expression by sine wave]

As shown in FIG. 14, an analysis range of a lung image is divided into four areas, whether change in signal of each area causes a difference or not is confirmed by the following method. That is, the correlation between each parameter and an FEV% estimation value is studied by approximating "change in signal: f(x)" to "g(x) = A * sin (Cx + B)".

As a result, as shown in FIG. 15 to FIG. 17, according to a healthy normal lung, there was a tendency that the amplitude became larger so as to be "R4 -> R3 -> R2 -> R1" and phases matched with each other. In contrast, in a COPD disease example, it is visually confirmed that not only amplitudes in size but also phases exhibit variations. It is determined by examining results of approximation to sine waves that phase shifting shows the FEV% estimation value and the negative correlation (R = -0.775). When taking the correlation, one point is excluded as an outlier, but it appears that this is caused by how to prepare a lung field (analysis range). In addition, as to the artifact of diaphragm and ribs, that mainly affects the amplitude, no tendency of contributing to phase shifting can be observed. When evaluating phase shifting thereof, it appears that at least it is not necessary to positively exclude R4/L4. On the other hand, when performing some estimation using an amplitude, it needs to be noted to receive influence on the artifact.

### [With regard to estimation of lung blood pressure]

In order to measure a lung blood pressure, the lung blood pressure is conventionally measured by inserting a catheter, but invasiveness appears high. In the invention according to the present application, though waveform data is taken from part of an organ, a sine wave is shown by averaging one part thereof. This sine wave is considered to be associated with the lung blood pressure. That is, it is made possible to calculate and determine a waveform of the lung blood pressure by an image analysis of the invention according to the present application, based on the waveform frequency, the phase, and the signal value. Herein, imaging is carried out by extracting cyclic movement matched with a heartbeat thereof. At this time, it is chased to change in matching with the blood flow in the image. For example, no sine wave is transmitted to the pulmonary hilum portion and the periphery thereof. Further, a delay is produced outward from a center of a lung. Thus, it is made possible to calculate a speed of the blood flow with this delay. The lung blood pressure can be estimated by size of blood vessels and the flow speed. Further, it is made possible to grasp disease conditions and cardiac failure as described in the present specification, such as blood vessel thickness, phase shifting, partial figures different from each other, respiration state, and so forth; and grasping of clinical conditions and disease levels in all or part of the disease. For example, it is made to contribute to a medical treatment policy of the cardiac failure by making AI learn a cardiac index and a numerical value of pulmonary artery wedge pressure or the like. An estimated respiration function can be calculated by calculating a level and a rate of respiration movement to superpose respiration coefficients on the outer side and the inner side of diaphragm from pulmonary apex. Further, according to matching a respiration function constant with a signal of blood flow or the like, a mismatch coefficient is measured, and it is made possible to estimate ventilation blood flow mismatch and ventilation blood flow imbalance, and a state (corresponding to lung disorder lung blood flow obstruction) of causing disorder, together with lung ventilation blood flow. That is, it becomes possible to be classified into pulmonary ventilation normality blood flow normality, pulmonary ventilation normality blood flow abnormality, pulmonary ventilation abnormality blood flow normality, and pulmonary ventilation abnormality blood flow abnormality, and it is made possible to determine the measure for grasping of the clinical condition and the medical treatment policy according to their degree.

The lung blood flow waveform calculated by the invention according to the present application is one obtained by digitizing a change rate in an arbitrary area as a graph, and the waveform of pulmonary artery recorded during actual right ventricle catheterization testing is approximate thereto. Pulmonary artery valve closure notch of a pulmonary artery pressure waveform or later corresponds to right ventricle diastole, and an angle until returning to the base line reflects lung blood vessel resistance. When using this feature, a PVR high value group and a low value group concerning the relationship between the lung blood flow waveform calculated by the invention according to the present application and a diastolic angle of an actual pulmonary artery pressure waveform, that is, a difference between a CpcPH patient and an IpcPH patient can be detected.

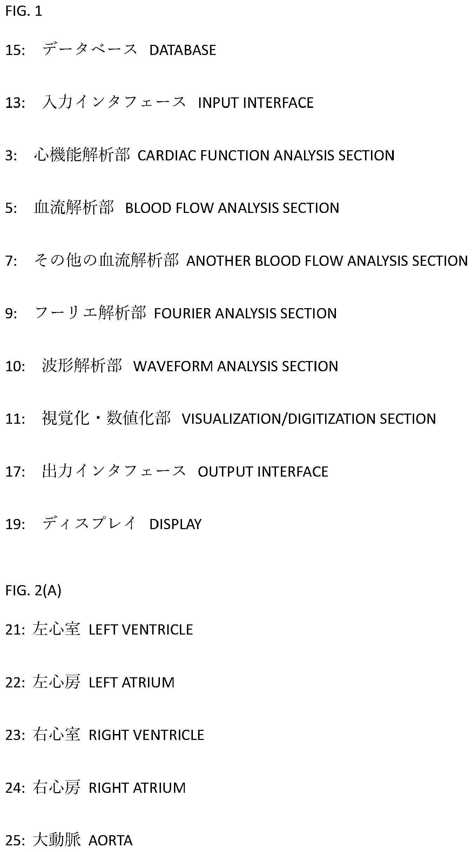

Next, an embodiment of the present invention is described referring to the drawings. FIG. 1 is a diagram showing an outline configuration of a diagnosis support system according to the present embodiment. This diagnosis support system performs a specific function by causing a computer to execute a diagnostic support program. A basic module 1 includes a cardiac function analysis section 3, a blood flow analysis section 5, another blood flow analysis section 7, a Fourier analysis section 9, a waveform analysis section 10, and a visualization/digitization section 11. The basic module 1 acquires image data from a database 15 via an input interface 13. The database 15 stores, for example, images via DICOM (Digital Imaging and Communication in Medicine). An image signal output from the basic module 1 is displayed on a display 19 via an output interface 17. Next, the function of the basic module according to the present embodiment is described. Furthermore, the input images are not limited to the database 15, but can also be input from other external devices via the input interface 13 or by initiative.

### [Refinement of detecting dynamic region]

There is a case where contrast in a dynamic region such as a lung field, a thorax, a heart, or the like is not uniform along a line. In this case, a shape of the dynamic region can be more precisely detected by changing a threshold value used for eliminating noises, and performing processing of detection plural times. For example, as to a left lung, the contrast on the line of the diaphragm tends to be weaker toward the inside of a human body. There is also a circumstance that the cardiac apex part and the cardiac base part each have smaller movement and the movement becomes larger at the heart center portion. In such a case, the remaining part of the left half of the diaphragm, and the region where the movement of the heart is large and the region where the movement of the heart is small can be detected by changing setting of the threshold value used for eliminating the noises, or by multiplying the pixel value by a different factor. It becomes possible to detect a shape of the whole diaphragm or to detect a shape of the whole heart by repeating this processing plural times. In this manner, the present method also enables digitizing not only the position of the diaphragm but also a change rate and a change amount of a line and a surface concerning a shape of a thorax, a heart, and a dynamic region, and it can be used for a new diagnosis.

In this manner, it becomes possible to utilize the position or shape of a diaphragm or a heart detected in a diagnosis. That is, it is possible to graph coordinates of the diaphragm and the heart; to calculate the coordinates of thorax, diaphragm, and heart using curves (plaques) or straight lines calculated as described above; and to graph a heartbeat, a blood vessel beat, "density" in a lung field and so forth as a position corresponding to a cycle, or coordinates. Such a method is also applicable to a dynamic region linked with respiration and cardiac pulsation.

When not only Hz in each of expiration, inspiration, a systole, and a diastole, but also a frequency of a dynamic region linked with diaphragm or respiration or a frequency (Hz) of a dynamic region of a heart is changed, such a method enables measurement in a frequency band responding to the change. Then, during spectrum extraction of BPF (band pass filter), in a fixed range, it becomes possible that BBF is set depending on each respective state of respiration or a heart; that an optimal state can be caused by variation of an axis at the BPF position in each "reconstructionphase" of respiration or a heart; and that BPF in variability accompanying the foregoing is prepared. Even when a respiratory rhythm varies so as to be at the time when breathing slow or stopping breathing (Hz=0), or even when temporary fibrillation of the heart (an extreme high frequency) or stopping breathing (Hz=0) appears, this enables providing images according to the foregoing.

In the present specification, as to "(A) a form of a wave itself", a concept of "waveform tunability" is used, and images are displayed based on this (wave form tunable imaging). Further, as to "(B) wave intervals (frequency: Hz)", a concept of "frequency tunability" is used, and images are displayed based on this (frequency tunable imaging).

For example, according to a heart, in the following description "an example of contrasting between a waveform of aorta blood flow quantity and a waveform of ventricular volume" as shown in FIG. 31A, the peak of the aorta blood flow quantity and the peak as well as the waveform of the ventricular volume are not identical to each other. However, when the temporal widths at equal intervals each such as time t1 to t2, time t2 to t3, time t3 to t4, and so forth as in FIG. 31A are determined as one cycle, one cycle of the aorta blood flow quantity and one cycle of the ventricular volume are repeated many times, and thus it can be said to be each waveform whose frequency is tuned. When attention is paid to this waveform, one cycle is specified from actual measurement values as shown in FIG. 31A, and a waveform (Wave form) can be predicted by using a model waveform. That is, as how to prepare "a waveform as base data", it may be actually measured; it may be produced from a frequency (cycle); it may be allowed by using the model waveform; and it may be used by averaging waveforms between individuals. When a cycle (period) of an organ having a frequency of a heart or the like is known, the waveform (Wave form) can be predicted, and thus waveforms such as the aorta blood flow quantity, the ventricular volume and so forth are grasped and it is made possible to display a dynamic image of the organ.

Further, in continuously imaged images, it is possible to detect movement of a dynamic region linked with a diaphragm or respiration. In the continuously imaged images, when images are selected at the arbitrary interval to calculate the difference between the images, an area exhibiting large contrast has the large difference. An area where movement occurs can be detected by appropriately visualizing this difference. During the visualization, the above-described waveform (wave form) can be prepared by emphasizing continuity of the areas having a large absolute value of the difference via curve fitting or the like in which noise elimination by a threshold value, a least squares method or the like is used. According to a lung field, the contrast on a line with which a diaphragm and a heart come into contact is outstanding, and as shown in FIG. 31B, when a difference in two lung images is taken to visualize the difference by setting a fixed threshold value, the line with which the diaphragm and the heart come into contact can be visualized, as shown in FIG. 31C.

Further, a frequency of the whole expiration or inspiration may be made to be calculated based on a ratio of a respiratory element (a respiratory element including all or part of expiration or inspiration) in the whole expiration or inspiration. Similarly, a systole or a diastole, a frequency element, and other whole frequencies may be made to be calculated based on a ratio of a cardiac dynamic element (cardiac dynamic element including all or part of the systole or diastole) in a cardiac systole and diastole, one pulsation of a heart, and pulsations of the whole measurement. In addition, a diaphragm and a heart are detected plural times, and one whose signal and waveform is stable may be made to be selected. Thus, it becomes possible to calculate at least one frequency of a respiratory element and a cardiac pulsation element from a position or shape of the detected diaphragm, or a position or shape of the dynamic region linked with a position or shape of the detected diaphragm and heart, or the respiration, and to calculate a frequency that represents the heartbeat. When the position or shape of the diaphragm and the heart or the dynamic region are/is able to be grasped, it becomes possible to grasp the respiratory element, the frequency of cardiac pulsation element, and the heartbeat. This method enables chasing the subsequent waveform even though dividing part of the waveform. Accordingly, it is possible to follow the original respiratory element or cardiac pulsation element even when the frequency of respiratory element or cardiac pulsation element changes on the way. Further, pulsation of a heart and so forth often undergo a sudden change, but the same thing is also possible to be applied to cardiac blood vessels and organs related to cardiac blood vessel waveforms.

### [Lang field detection]

In the present invention, it is possible to refine lung field detection as one aspect of the above-described "refinement of detecting a dynamic region". In this processing, after setting the maximum and minimum lung fields, the other lung fields are calculated using values thereof. FIG. 11 is a diagram showing one example of a method of detecting a lung field according to the present invention. In this method, the "B-spline curve" is used to represent the "coefficient of each image". In FIG. 11, the waveform X represents the "coefficient of each image L showing the lung field", and from the left to right direction, the coefficient of the first image, the coefficient of the second image... and so forth are shown. When the control point Y in FIG. 11 is moved, the "coefficient of each image" changes smoothly. In the present invention, in this manner, the graph of the coefficients can be edited directly. In FIG. 11, the "gray polygonal line Z" represents the "pixel average value of each image". When imaging under optimal conditions, the change in the size of the lung field is identical to the change in the pixel average value. Herein, this pixel average value can be smoothed by curve fitting and also directly used as a "coefficient". The same method can be applied to a heart and others such as organs in relation with a frequency of cardiac blood vessels.

### [Cardiac function analysis]

FIG. 2 is a cross-sectional view showing an outline configuration of a heart. The "cardiac function" is generally defined as "the pumping function of the left ventricle, which circulates blood through the body". Cardiac function analysis is important for estimating the prognosis of patients with "ischemic heart disease", especially "myocardial infarction". For example, when the value of the left ventricular ejection fraction (EF) decreases, the heart's output as a pump decreases, and it becomes unable to pump enough blood throughout the body. Additionally, cardiac functions include left ventricular end-diastolic volume (EDV), left ventricular end-systolic volume (ESV), stroke volume (SV), cardiac output (CO), and a cardiac index (CI). For local evaluation of myocardium, "Bull's eye map" showing wall thickness, wall movement, a change rate of wall thickness, or the like is used, as shown in FIGS. 2B and 2C each that are cross-sections in planes perpendicular to the axial line A in FIG. 2A. This "Bull's eye map" is an image displayed with the cross-section of the cardiac apex part placed in the center of the circle so as to outwardly arrange the short-axis tomographic images in a concentric-circular shape in order, and to arrange the cross-section of the heart base part on the outermost side.

According to the present embodiment, in addition to the "Bull's eye map" being used, the cycle of heart movement is analyzed based on the following indexes. That is, the cycle of the heart movement is analyzed by using density/intensity in a fixed area inside a cardiac area. Further, a range constituted from certain fixed volume density/intensity measured in a region exhibiting high permeability of X-rays (besides that, a plurality of kinds of modality such as CT and MRI), data obtained by another measurement method such as spirogram or the like, and external input information may also be used. In addition, it is desirable to compare the analysis results for each heartbeat and analyze the tendency from a plurality of pieces of data to improve the accuracy of the data. Further, it is also possible to identify the edge of a heart and obtain a frequency based on a change in this edge of the heart. Further, it is also possible to identify the side edge of a lung field and obtain a frequency from the movement of this side edge.

### [Blood vessel beat analysis]

According to the present embodiment, the blood vessel beat is analyzed based on the following indexes. That is, the blood vessel beat is analyzed using a change in density/intensity of each region by specifying the heart/ pulmonary hilum position/main blood vessels from the measurement results of other modalities such as an electrocardiogram and a pulsimeter, or from the lung contour. Further, a change in density/intensity of a target region may be analyzed by manually performing plotting on an image. Then, it is also possible to use the heartbeat element obtained from a heartbeat or a blood vessel beat. In addition, it is desirable to improve accuracy of data by comparing the analysis result for every beat and analyzing a tendency from a plurality of pieces of data. Further, it becomes possible to improve accuracy by performing the extraction of density/intensity of each region plural times, as well as by performing the foregoing with respect to a fixed range. Further, there is also a method of inputting a cardiovascular beat frequency or a frequency band.

### [Identification of cardiac area]

An image is extracted from the database (DICOM), and a cardiac area (specifically myocardium) is automatically detected by using the cardiac function analysis result as described above. Next, the myocardium is divided into a plurality of block areas to calculate a change in each block area. Herein, size of the block area may be determined depending on an imaging speed. When the imaging speed is low, the corresponding area in the frame image next to a certain frame image is difficult to be specified, and thus the block area is made to be large. On the other hand, when the imaging speed is large, the number of frame images per unit time is large, and thus, it becomes possible to follow even when the block area is small. Further, the size of the block area may be calculated depending on which timing out of a cycle of the cardiac movement is selected. Herein, there are some cases where deviation of the myocardial area needs to be corrected. In this case, the cardiac movement is identified, and the relative position of the cardiac contour is further grasped to be relatively evaluated based on the movement. In addition, when the block area is too small, a flicker often occurs in the image. In order to prevent this, the block area needs to have a fixed size.

### [Preparation of block area]

Next, a method of dividing myocardium into a plurality of block areas is described. FIGS. 2B and 2C each are a diagram showing the method of dividing myocardium from radially the center of a heart. As to the cardiac area, the positional relationship between movement of the heart and blood vessels is identified and the relative position of the cardiac contour is grasped, and the evaluation should be relatively made based on the movement. Thus, in the invention according to the present application, after automatically detecting a cardiac contour, a myocardial area is divided into a plurality of block areas to average the value (pixel value) of a change of an image included in each block area. As a result of this, even when a form of a heart changes over time, it becomes possible to chase a temporal change of the area of interest.

On the other hand, when being divided into block areas without specifying a cardiac area, the region of interest falls outside the cardiac area due to a temporal change of the heart, thereby resulting in a meaningless image. Further, there is also provided a method of inputting a heartbeat or a frequency band. Further, these methods are also applicable to three-dimensional stereoscopic images. By making pixels in 3D stereoscopic images be constant, area-dividing calculation can also be made for 3D. In addition, the relative evaluation based on the movement of such relative positions can be made between frame images adjacent to each other, respectively or can be made for every integer multiple thereof, such as every two images or every three images. Further, several images are collectively put, and processing may be performed for every set of the images that are collectively put.

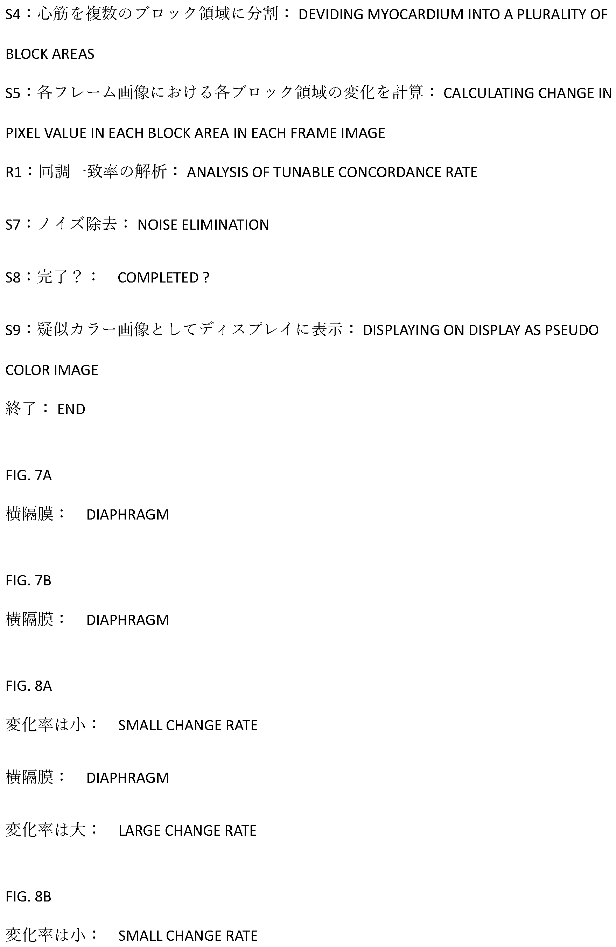

FIG. 7A is a schematic diagram showing a left lung of a human body from the front, and FIG. 7B is a schematic diagram showing a left lung of a human body from the left side face. FIGS. 7A and 7B both show the inspiration, that is, a lung in a breathing-in state. FIG. 8A is a schematic diagram showing a left lung of a human body from the front, and FIG. 8B is a schematic diagram showing a left lung of a human body from the left side face. FIGS. 8A and 8B both show the expiration, that is, a lung in a breathing-out state. As shown in these figures, the form of a lung field changes largely during respiration, but the change rate of the lung field on the diaphragm side is large, while the change rate of the lung field on the opposite side of the diaphragm is small. In the present invention, the position of each region in the lung field is changed depending on this change rate. This enables performing relative evaluation based on the relative positional relationship of each area within the lung field area. In addition, based on the change rate in the lung field (for example, average change rate), the relative positional relationship can be represented by a fixed change rate in the lung field area, or the change rate may be adaptively varied in the lung field area depending on the distance from the diaphragm. In this manner, it becomes possible to display an image synchronized with the respiratory cycle by using the variation rate in the lung field area.

FIG. 9A is a schematic diagram showing a left lung of a human body from the front, and FIG. 9B is a schematic diagram showing a left lung of a human body from the left side face. FIGS. 9A and 9B both show the inspiration, that is, a lung in a breathing-in state. FIG. 10A is a schematic diagram showing a left lung of a human body from the front, and FIG. 10B is a schematic diagram showing a left lung of a human body from the left side face. 10A and 10B both show the expiration, that is, a lung in a breathing-out state. For example, as shown in FIGS. 9A and 9B, marker PI is plotted in a certain place within the lung field area in a state of inspiration. Assuming that the marker PI is a fixed point as provided by two-dimensional coordinates, the coordinates remain unchanged even in the expiration state, and thus as shown in FIGS. 10A and 10B, the marker PI exists at the same position. On the other hand, in the present invention, as described above, the evaluation is made by the relative positional relationship with respect to the whole lung field area, thereby resulting in movement to a position of marker P2 instead of the position of the marker PI in the expiration state. It is also possible to evaluate the point plotted during inspiration and the point obtained by identifying a moving destination during expiration, using the vector at this time.

When dividing a region thereinto, Voronoi tessellation (Thiessen tessellation) is applicable. As shown in FIG. 2D, the Voronoi tessellation is "a method of drawing perpendicular bisectors each on the straight line connecting neighboring base points to divide the nearest neighbor area of each of the base points thereinto". It becomes possible to shorten the calculation time by applying such Voronoi tessellation thereto. Further, when drawing a straight line connecting the neighboring base points, weighting may be applied according to the analysis target. For example, when dividing a pulmonary artery area, the thicker area may be weighted high and the thinner area may be weighed low. This enables carrying out the division according to the analysis target while reducing the processing burden. In addition, as to a plurality of block areas generated by the division, classification processing may be carried out based on indicators such as a change in pixel value (cyclic change).

In this manner, after dividing an area into a plurality of block areas, a change of an image in each block area is calculated based on the relative position of each block area to a dynamic region such as a heart. Herein, not only the signal difference is taken with only the range of the mass itself as to a pixel as one unit, but also the signal difference may be taken in a range smaller than the mass, or in a larger range so as to be larger than the mass, and to surround the periphery. Further, it may also be allowed to make only the range in the upward-downward direction larger in the vicinity of the diaphragm; to make only the range in the right and left direction larger in other dynamic regions; to deform the shape of the range; and to connect pixel areas. Further, after calculating one or more differences, it is desirable to define the form of the mass again in association with the whole lung field and the form of a heart. For example, after subjecting the first image to the second image to processing, the second image to the third image may be compared therewith by further preparing and matching the form of the mass with the shape one more time with the second image.

In the above-described description, the "relative positional relationship" in consideration of the organ movement is described, but the present invention is not limited to this and it is also possible to perform image processing while maintaining "absolute positional relationship of each pixel" in a plurality of frame images. The "absolute positional relationship of each pixel" means the relationship of pixel-to-pixel having coordinates specified based on the two-dimensional coordinate axis, when the two-dimensional coordinate axis is defined on the frame image. That is, it is a method of pixel-processing with the pixel of interest as being invariant. The processing in a state where the "absolute positional relationship of each pixel" is maintained is premised on a plurality of frame images, but the number of frame images is not specified. A plurality of frame images can be classified into a plurality of groups, and the equal number of frame images may be included in each group, or the different number of frame images may be included in each group.

That is, a plurality of frame images are classified into a plurality of groups, and a change in pixel value of an organ, a change in distance from the center of the organ to an outer edge thereof, or a change in volume of the organ is calculated in the state where the absolute positional relationship of each pixel inside a plurality of frame images belonging to each group is maintained. This enables handling the pixel value as being even, even when the pixel value varies in some degree, thereby being able to reduce the data amount and processing steps.

Further, the positional relationship that is neither relative positional relationship nor absolute positional relationship can be conceived. That is, one point P having specific coordinates in a specific frame image is defined, and the other point Q. having different coordinates from the above specific point can be defined inside the next and succeeding frame images, but in this case, the size of vector PQ. is made to be small with respect to the movement of the organ. Further, for the point Q, another point R having slightly different coordinates from those of the point Q is defined in the next and succeeding frames. By repeating this operation, the other point having a slight deviation from a specific point P is extracted for every frame image, and the present embodiment is applied thereto. Specifically, a plurality of frame images are acquired, and for each pixel inside a specific frame image, pixels having different coordinates from each of the pixels inside the next and succeeding frame images is extracted, and a cyclic change characterizing the state of the organ is calculated.

Then, the cyclic change characterizing the state of the organ is subjected to Fourier-transforming; a spectrum in a fixed band including a spectrum corresponding to a frequency of movement of an organ out of a spectrum obtained after the Fourier-transforming is extracted; and the spectrum extracted from the fixed band is subjected to inverse Fourier transform to output each of the images after performing the inverse Fourier transform. Further, pixels that change according to a frequency of a cyclic change characterizing the state of the organ in the image are extracted by a digital filter, and images including the pixels extracted by the digital filter may be output. Further, the color corresponding to the cyclic change rate characterizing the state of the organ is selected, and the image may be displayed on a display by adding the selected color to the change rate of the pixel value. This enables expressing the movement of the organ.

Next, artifacts are eliminated to interpolate the image data. That is, when bones and so forth are included within the analysis range, noises are generated, and thus, it is desirable to eliminate the noises, using a noise-cut filter. As to X-ray images, conventionally, air and bones are set to-1000 and 1000, respectively, and thus a high permeability portion exhibits a low pixel value, thereby being displayed black, and a low permeability portion exhibits a high pixel value, thereby being displayed white. For example, when displaying the pixel value by 256 gradations, black becomes 0, and white becomes 255.

Inside a cardiac area, X-rays hardly transmit the position where blood vessels and bones are present, and thus the pixel value of an X-ray image becomes high and the X-ray image becomes white. The same thing can also be additionally applied to CT and MRI. Herein, from the result of the above-described cardiac function analysis, it becomes possible to eliminate artifacts by interpolating data, using values of the same phase, based on a waveform per heartbeat. Further, when detecting that "coordinates are different therefrom", "the pixel value extremely varies", or "a frequency and density abnormally become high", cut-off is carried out for these, and it is made possible to be used for Hz calculation of the heart movement and adjustment of the myocardial area by identifying a continuously smooth waveform using a least squares method or the like, for example, with respect to the remaining resulting images. Further, when images are superposed, there are (1) a case where it is made to superpose them while acquired comparison images obtained by acquiring the image of one side before or after are kept at the coordinates, and (2) a method of superposing relative positional information on a base by relatively extending the image, after acquiring the image of one side before or after onto the base. It becomes possible to correct a form of a cardiac area, or to correct an image change in the block area by using each of the above-described methods.

Herein, "reconstruction" in the time axis is described. For example, when inspiration time of 15 f/s is 2 seconds, 30+1 images are obtained. In this case, the "reconstruction" for each 10% can be carried out when 3 images are simply superposed at each time. At this time, for example, when 0.1 seconds indicate 10%, and the image acquires only pictures of 0.07 seconds and 0.12 seconds, "reconstruction" of 0.1 seconds is needed. In this case, the "reconstruction" is carried out by providing an intermediate value in image of about 10%, a value (obtained by averaging both). Further, it is taken by the time axis, and the coefficient may be changed at a ratio of the time. For example, when there is the time axis difference, and there is an imaging time of each of 0.07 seconds and 0.12 seconds with no imaging value of 0.1 seconds, "reconstruction" can be made via recalculation as "(a value of 0.07 seconds thereof) × 2/5 + (a value of 0.12 seconds) × 3/5". In addition, it is desirable to include 0 to 100% of "Maximum Differential Intensity Projection", and to perform calculation by providing a range such as "reconstruction" of 10 to 20%, "reconstruction" of 10 to 40%, or the like. In this manner, it is possible to carry out "reconstruction" at a ratio of one heartbeat for the non-imaged portion as well. In addition, according to the present invention, it is also possible to carry out "reconstruction" in the same manner for a heart and a blood flow, and a series of movements linked with the foregoing as well in addition.

### [Fourier analysis]

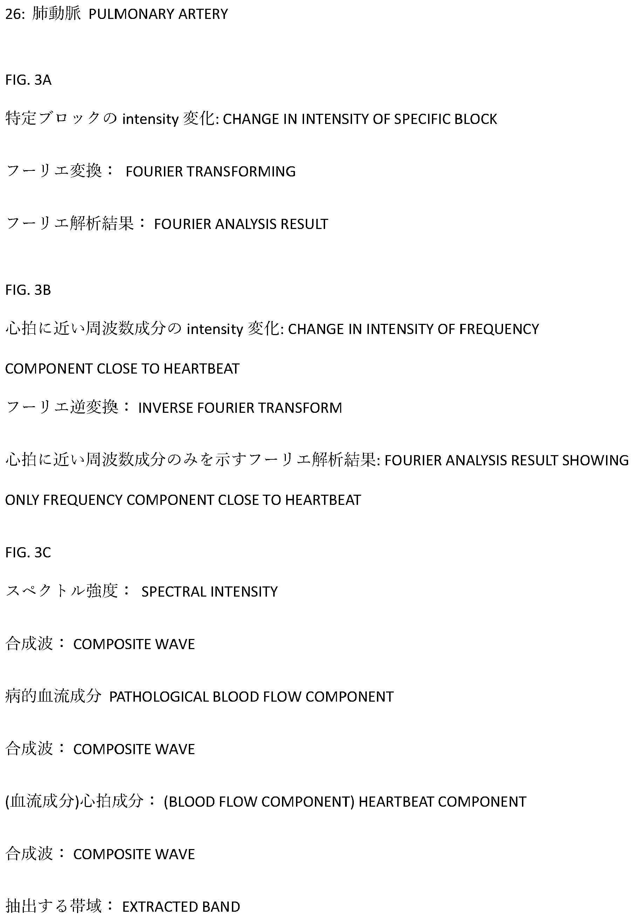

Based on the cardiac movement cycle and the blood vessel beat cycle that are analyzed as described above, the value of density/intensity in each block area and a change amount thereof are subjected to Fourier analysis. FIG. 3A is a diagram showing a change in intensity of a specific block and a result obtained by performing Fourier analysis thereof. FIG. 3B is a diagram showing a Fourier transform result obtained by extracting a frequency component close to a heartbeat and a change in intensity of the frequency component close to the heartbeat, that is obtained by performing inverse Fourier transform on this. For example, when the change in intensity of a specific block is subjected to Fourier-transforming (Fourier analysis), the results as shown in FIG. 3A are obtained. Then, the results as shown on the right side with respect to the drawing face of FIG. 3B are obtained by extracting the frequency component close to the heartbeat from the frequency components as shown in FIG. 3A. By performing inverse Fourier transform on this, the change in intensity, that is tuned to the heartbeat change can be obtained as shown on the left side with respect to the drawing face of FIG. 3B.

Herein, when the spectrum including frequency components is subjected to inverse Fourier transform, the inverse Fourier transform may be performed by using both a frequency element (a heartbeat, and a cardiovascular beat frequency) specified from density of the heartbeat and the blood flow, and a spectrum band (BPF may be used) in addition; or based on either of those elements.

In addition, when executing Fourier transform, it is possible to use an AR method (Autoregressive Moving average model) so as to perform calculation in a short period of time. According to the AR method, there is a method of using a Yule-walker equation (Yule-walker equiation) or a Kalman filter in an autoregressive moving average model, and it is possible to make up for the calculation by using a Yule-walker estimation value (Yule-walker estimates) derived therein, a PARCOR method, or a least squares method. This enables acquiring a near-real-time image, assisting the calculation, and correcting the artifact at a higher speed. It becomes possible to extract and display the image property in each block area via such Fourier analysis.

Herein, a spectrum within a fixed band including a spectrum corresponding to a cardiac movement cycle, out of a spectrum obtained after Fourier-transforming can be extracted by subjecting an image change in each block area in each frame image to Fourier-transforming. FIG. 3C is a diagram showing an example of extracting a certain fixed band out of a spectrum obtained after Fourier-transforming. As to a frequency f of a composite wave spectrum, the relationship of "1/f = 1/f1 + 1/f2" is satisfied between f1 (a heartbeat component) and f2 (a pathological blood flow component) as each frequency that becomes a composite source, and when extracting the spectrum, it is possible to employ the following method.

(1) A portion having a high spectral ratio of heartbeat is extracted.
(2) A spectrum is extracted by being separated midway between the peak of a spectrum corresponding to a heartbeat/a pathological blood flow and the peak of a plurality of composite waves in the vicinity thereof.
(3) A spectrum is extracted by being separated by the peak of a spectrum corresponding to a heartbeat/a pathological blood flow and the valley portion of the spectrum of a plurality of composite waves in the vicinity thereof.
(4) A spectrum included in a certain fixed bandwidth may be extracted from a heartbeat component (blood flow component). In this case, a spectrum obtained by superposing a plurality of spectra is acquired, but it is made possible to recover each spectrum by separating each component.

As described above, according to the present invention, it does not mean that a fixed BPF is used, and a spectrum in a fixed band including a spectrum corresponding to a cycle of a cardiac movement is extracted. Further, in the invention according to the present application, it is possible to extract a spectrum in a fixed band including a spectrum (for example, spectral model) corresponding to a frequency (for example, further, a heartbeat element obtained from density/intensity, a heartbeat, or a blood vessel beat in each region) other than a frequency of a cardiac movement obtained from a frame image, or a frequency input from outside by an operator, out of a spectrum obtained after the Fourier-transforming.

Herein, a composition of a composite wave spectrum when comprising only two components (heartbeat and pathological blood flow) becomes 50% + 50%, and whose one-third each is distributed when comprising three components. Thus, the composite wave spectrum can be calculated to some extent from the spectral component and its height such as what percentage the heartbeat component spectrum is and what percentage the pathological blood flow component spectrum is. It is possible to extract the spectrum at a high ratio (%) thereof. That is, a ratio of pathological blood flow component/heartbeat component to a composite wave component is calculated, and a spectral value having high pathological blood flow component/heartbeat component is calculated and extracted. In addition, as to identification of diaphragm or the like, there are some cases where only a spectrum or superposition thereof corresponding to a region in which Hz (frequency) becomes relatively constant, that is, the area in which a change in Hz is small is extracted from data obtained by acquiring frequencies of the heartbeat and the heart blood vessels. Further, when determining a spectral band, there are also some cases of determining the spectral band in a range where a change in Hz is generated, and in its peripheral area. Therefore, it becomes possible to also extract the spectrum that should be taken into account, in addition to the case of being exactly identical to the cardiac movement cycle or the blood vessel beat cycle, thereby being able to contribute to an image diagnosis.

In addition, it is known that a "heartbeat" and "respiration" are included in a specific frequency band. Accordingly, by using a filter of, for example, "0 to 0.5 Hz (a respiratory rate of 0 to 30 times/min)" in the respiration case, and of, for example, "0.6 to 2.5 (a heartbeat/pulse rate 36 to 150 times/min) Hz" in the circulatory system case, it is possible to previously specify a respiratory frequency and a circulatory system frequency with this filter. This enables displaying a frequency tunable image. This is because there are cases where a change in density of respiration (lung) is picked up when acquiring a change in density of a heart, and a change in density of the heart is picked up when acquiring a change in density of a lung.

### [Waveform analysis]

A heart, blood vessels, brain waves, and others recognized as constant waveforms by examinations are subjected to waveform analysis. The movements repeated in a constant state such as foot movements are included. Further, whether or not to exhibit the same tendency by superposing Hz of the action repeatedly carried out is analyzed. The concordance rate between two pieces of data is calculated in comparison to the waveform data. Then, the data after Fourier analysis is compared therewith.

### [Digital filter]

In addition, a digital filter is usable in place of the above-described Fourier analysis. The digital filter is made possible to convert the time domain into the frequency domain, based on the "fast Fourier transform and inverse fast Fourier transform" as mathematical algorithms for extracting frequency components of a signal in order to adjust the frequency components of the signal. This enables acquiring the identical effect to the above-described Fourier analysis.

### [Visualization/digitization]

The result analyzed as described above is visualized and digitized. As standard uptake, the value is often displayed relatively/logarithmically with the average value as 1 from density/intensity in the whole area of a measured heart. Further, only the blood flow direction is employed, and thus a change to a specific direction is often cut out. This enables taking out only the data in a significant method. The pseudo colorization is carried out following a change in an analysis range, using the identification result of the cardiac area. That is, the analysis result of each individual (subject) is fitted to a relative area along a specific shape (minimum, maximum, mean, median) adapted with the phase. Further, a plurality of analysis results are made to be deformed to a comparable specific shape/phase.

Further, when preparing a "standard heart", the relative positional relationship within the heart (myocardium)is calculated, using the result of analyzing movement of the above-described heart. In addition, the "standard heart" is prepared, using a line obtained by collectively averaging contour lines, density, or the like of a plurality of patient' hearts. In addition, the foregoing way of thinking is not limited to a heart, but can be applied to lungs (standard lungs) and other organs (standard organs). For example, it is possible to separately prepare an "organ model" by age, gender, country, and a disease degree.

It is also possible to perform Fourier analysis by calculating a change in distance (distance L shown in FIGS. 2B and 2C) from the center of a heart to the myocardium in addition to a change in pixel value of the heart as described above, and further, it is possible to perform the Fourier analysis by calculating a change in volume of the heart.

When "standard heart" is able to be prepared, it becomes possible to digitize and present tunability, a concordance rate, and a non-concordance rate (displaying of a frequency tunable image). Further, deviation from a normal state can be displayed. According to the present embodiment, discovery of possibility of a new disease, comparison with oneself in the normal state, comparison of one hand with one foot, and comparison between the other hand and the other foot are made possible by executing Fourier analysis. Further, it becomes possible to grasp where is abnormal with respect to how to move the foot, deglutition or the like by digitizing the tunability. Further, whether or not a person in the disease state changes therein after a fixed time elapses is determined, and further when changing therein, those before and after the change become comparable.

### [Drawing of heart]

In the present specification, a method of adjusting a heart is employed so as to exhibit a high matching property by temporarily drawing a contour of the heart by using Bezier curves and straight lines in combination. For example, when a contour of the heart is expressed with 4 Bezier curves and one straight line, it becomes possible to draw the contour of the heart by finding 5 points on the contour of the heart and 4 control points. It becomes possible to detect the contour of the heart with high accuracy by displacing the point position to draw a plurality of the contour of the heart, and evaluating a matching property, using a condition under which "the total value of density inside the contour becomes maximum"; "the difference of the sum in density for a few pixels inside and outside the contour line becomes maximum"; or the like. In addition, it is also possible to extract the point near an outer edge by extracting the contour via classical binarization, and to adjust the control point position of the Bezier curve by using a least squares method or the like. Further, the above-described method is not limited to a heart, but is also applicable to other organs as "organ detection". Further, it is applicable to not only planar images but also stereoscopic images (3D images). It becomes possible to estimate that a target object surrounded by a plurality of curved surfaces is an organ, by defining a curved surface equation to set the control point thereto.

### [Cardiac function analysis using Fourier analysis]

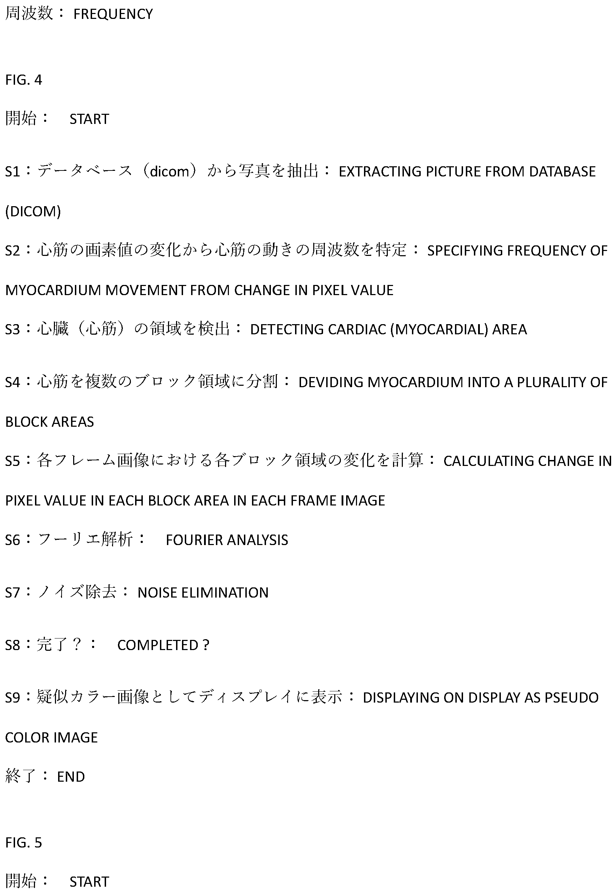

Next, the cardiac function analysis using Fourier analysis according to the present embodiment is described. FIG. 4 is a flowchart showing an outline of cardiac function analysis according to the present embodiment. The basic module 1 extracts images of DICOM from the database 15 (step SI). Herein, a plurality of frame images included within at least one heartbeat are acquired. Next, in each acquired frame image, the cycle of the heart movement is specified by using a change in pixel value, for example, a change in density (density/intensity) in at least a certain fixed area of the myocardium (step S2). Next, the cardiac (myocardial) area is detected (step S3), and the detected myocardium is divided into a plurality of block areas (step S4). Herein, as described above, the myocardium is radially divided from the center of the heart, using Voronoi tessellation (Thiessen tessellation). Then, a change in pixel value in each block area in each frame image is calculated (step S5). Herein, change values within each block area are averaged and expressed as one piece of data.

In addition, it is also possible to vaguely display the pixel to a certain extent, and to display the whole by making it into a dimmed state. Specifically, in the case of blood vessels, a low signal value coexists between high signal values, but if only the high signal values can be roughly grasped, it is acceptable to be vague as a whole. For example, in the case of blood flow, only a signal having a threshold value or more may be extracted. Specifically, in the case where a numerical value in the center is taken with the numerical value in the following table as one pixel, when a ratio occupied by the numerical value in the center is acquired and averaged within one pixel, the expression thereof can be smoothly made between neighboring pixels.

**TABLE 1**

| |
|---|
| 111 |
| 121 |
| 111 |

In addition, as to the change value within each block area, the noise elimination may be carried out by cut-off. Next, the value of density/intensity in each block area and a change amount thereof are subjected to Fourier analysis, based on the cycle of the above-described cardiac movement (step S6). This enables extracting and displaying an image property in each block area.

Herein, a spectrum in a fixed band including a spectrum corresponding to a cycle of the heart, out of a spectrum obtained after Fourier-transforming can be extracted. Herein, as to a frequency f of a composite wave spectrum, the relationship of "1/f = 1/f1 + 1/f2" is satisfied between f1 and f2 as each frequency that becomes a composite source, and when extracting the spectrum, it is possible to employ the following method.

(1) A portion having a high spectral ratio of heart movement is extracted.
(2) A spectrum is extracted by being separated midway between the peak of a spectrum corresponding to heartbeat/blood flow and the peak of a plurality of composite waves in the vicinity thereof.
(3) A spectrum is extracted by being separated by the peak of a spectrum corresponding to heartbeat/blood flow and the valley portion of the spectrum of a plurality of composite waves in the vicinity thereof.

Herein, a composition of a composite wave spectrum when comprising only two components (heartbeat and blood flow) becomes 50% + 50%, and whose one-third each is distributed when comprising three components. Thus, the composite wave spectrum can be calculated to some extent from the spectral component and its height such as what percentage the heartbeat component spectrum is and what percentage the blood flow component spectrum is. It is possible to extract the spectrum at a high ratio (%) thereof. That is, a ratio of blood flow component/heartbeat component to a composite wave component is calculated, and a spectral value having high blood flow component/heartbeat component is calculated and extracted.

Next, noise elimination is carried out for the result obtained by the Fourier analysis (step S7). Herein, the cut-off as described above and elimination of artifacts can be eliminated. Operations from step S5 to step S7 as described above are carried out at least once, and whether or not to be completed is determined (step S8). When not being completed, transitioning to step S5 results, but when being completed, the result obtained by the Fourier analysis is displayed on a display as a pseudo color image (step S9). In addition, black and white images may be displayed. There are some cases where data accuracy is enhanced by repeating a plurality of cycles in this manner. Thus, it becomes possible to display a desired moving image. Further, the desired moving image may be obtained by correcting the image displayed on the display.

In addition to the division processing of the myocardium in steps S4 and S5 as described above, it is also possible to perform Fourier analysis by calculating a change in distance from the center of the heart to the myocardium in place of the steps S4 and S5. Further, it is also possible to perform Fourier analysis by calculating a change in heart volume in place of the steps S4 and S5.

### [Cardiac function analysis using digital filter]

Next, cardiac function analysis using a digital filter according to the present embodiment is described. FIG. 5 is a flowchart showing an outline of the cardiac function analysis according to the present embodiment. Step S1 to step S5, and step S7 to step S9 are identical to those of the "Cardiac function analysis using Fourier analysis" as described above, and thus explanation therefor is omitted. In step T1 in FIG. 5, digital filter processing is performed (step T1). The digital filter is one performing conversion between the time domain and the frequency domain based on the "fast Fourier-transforming and inverse fast Fourier transform" that are mathematical algorithms for extracting frequency components of a signal in order to adjust the frequency components of the signal. This enables acquiring the same effect as the above-described Fourier analysis.

### [Cardiac function analysis using tunable concordance rate]

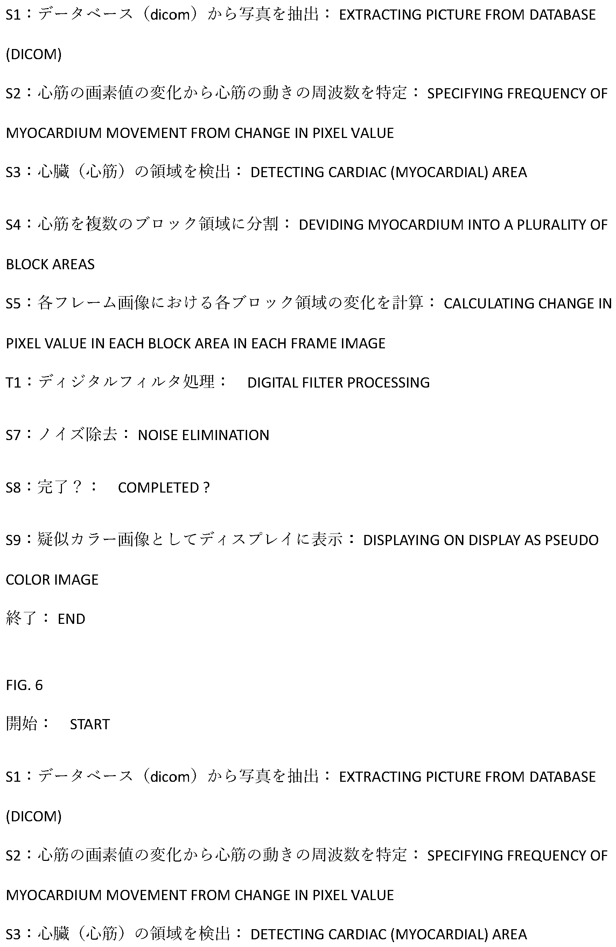

Next, cardiac function analysis using a tunable concordance rate according to the present embodiment is described. FIG. 6 is a flowchart showing an outline of the cardiac function analysis according to the present embodiment. Step S1 to step S5, and step S7 to step S9 are identical to those of the "Cardiac function analysis using Fourier analysis" as described above, and thus explanation therefor is omitted. In step R1 in FIG. 6, analysis of a tunable concordance rate is carried out (step R1). That is, the cardiac (myocardial) area is detected (step S3); and after the myocardium is divided into a plurality of block areas (step S4), average density (pixel value x) of a block area in each frame image is calculated, and a ratio (x') of an average pixel value of the block area in each frame image to a change width (0% to 100%) from the minimum value to the maximum value of the average density (pixel value x) is calculated (step S5). On the other hand, a ratio value (x'/y') obtained by using a ratio (y') of a heart change (y) in each frame image to a change width (0% to 100%) from the minimum to the maximum of a surface area (or volume) of a heart is calculated (step S5). By using them, only the block area where the ratio value (x'/y') falls within a predetermined fixed range can be extracted (step R1).

Herein, the case where y'=x' or y=ax (coefficient of a numerical value of amplitude of a surface area or volume of a heart, or a numerical value of density is represented by a) means complete concordance. However, only the case of the complete concordance does not mean a meaningful value, and a value having a certain fixed width should be extracted. Thus, according to one aspect of the present invention, the fixed width is determined as described below, using logarithms (log). That is, when being calculated at a ratio (%) where y=x, the complete concordance of tunability is "log y'/x'=0". Further, when extracting one in which a range of a tunable concordance rate is narrow, or a (numerically narrow) range, for example, it is defined as "log y'/x'=-0.05 to +0.05" in the range near 0; and when being one in which the range of the tunable concordance rate is wide, or a (numerically wide) range, for example, it is defined as "log y'/x'=-0.5 to +0.5" in the range near 0. As this range is narrower, or the numerical value identical thereto becomes higher within the range, the concordance rate is said to be higher. When counting the number by finding this ratio value for every pixel of the pixels, a normal distribution with a complete concordance case as a peak is obtained in the case of a healthy person's heart. In contrast, in the case of those each having disease, the distribution of this ratio value becomes collapsed. In addition, as described above, the method of determining a width using logarithms is only one example, and the present invention is not limited thereto. That is, the present invention is one performing "image extraction" as the following: (average of a change in density in a certain approximate range) ≈ (a heartbeat) ≈ (a change in heart) ≈ (an electrocardiogram) ≈ (a change in surface area and volume of a heart), and methods other than the method of using logarithms are also applicable.

In addition, when being considered in 3D, by measuring a heartbeat, a surface area or volume of a heart, cardiac output, and a central blood flow amount with an extra device, it becomes possible to measure the "partial heart surface area", "partial heart volume", and "blood flow rate" in each area from these rates. As these quantitative measurements, when it becomes possible to measure a heart surface area, heart volume, cardiac output, and blood flow on the center side with another modality or the like, it becomes possible to estimate the estimated functional amount from quantity of one frame and a rate thereof, or a change amount rate in the area. That is, in the case of cardiac function analysis, it becomes possible to estimate the heart volume from heart movement; and in the case of blood flow analysis, it becomes possible to estimate a lung blood flow amount from the cardiac output, and to estimate an estimated blood flow amount (rate) in bifurcated blood vessels depicted from the blood flow amount (rate) on the center side.

Further, as described above, according to the acquired database, it is possible to be determined with higher accuracy if being able to be wholly calculated, but time is often required when computer analysis is executed. Thus, it can be made possible to perform calculation by extracting only a certain fixed number (phase) thereof. For example, the acquisition is automatically made with images not from the beginning of the acquired images, but from the latter half of the images, that is, from with the rear of the middle portion as a center, that is not going till the end (the acquisition may be manually made). This makes it easier to extract more stable images by cutting out imaging in a tense state generated for the first time, when imaging a patient. Further, a turning point in a "heart movement" can be selected at the position of the heart measured for the first time or the like, and subsequently calculated with no calculation of the acquired images (for example, 300 images) as they are. This enables image labeling that looks like a continuous heartbeat when moving image labeling or the like needs to be repeatedly viewed. In this case, calculation can be made by image labeling. In addition, when identifying the cardiac (myocardial) area, even when manually changing shape of only part thereof, or manually changing only part of the graph labeling, it is desirable to correct the graph by using noise cutting and a least squares method.

As described above, according to the present embodiment, it becomes possible to evaluate images of a human body with an X-ray moving image device. If digital data can be obtained, it is possible to be calculated with a currently existing facility device in a generally excellent manner, and thus installation cost is reduced. For example, according to the X-ray moving image device using a Flat panel detector, it becomes possible to simply complete the examination of a subject. In cardiac function analysis, screening of myocardial infarction becomes possible. For example, according to the X-ray moving image device using the Flat panel detector, useless examinations can be eliminated by executing a diagnostic support program according to the present embodiment before performing CT. Further, the examination is simply carried out, and thus it becomes possible to quickly find a highly urgent disease and respond to it on a priority basis. In addition, according to the imaging method at the current time, another modality such as CT, MR or the like has produced several problems, but a detailed diagnosis in each area becomes possible if the foregoing can be solved.

Further, it is also applicable to screening of various kinds of blood vessels, for example, cervical blood flow narrowing; and is also applicable to the blood flow evaluation and screening of large blood vessels. Further, it is also possible to be applied for grasping characteristic conditions before and after operation. Further, an abnormal change of the remaining biological movement, for example, intestinal tract ileus or the like can be observed by Fourier-transforming a cycle of heart movement and a blood flow cycle, and eliminating a waveform of the heart movement and a blood flow waveform on an X-ray image of an abdomen.

In addition, when an initially acquired image exhibits high resolution to a certain extent, the number of pixels is large, and thus it often takes time for calculation. In this case, the calculation may be made after reducing the image to a fixed number of pixels. For example, it is possible to suppress the calculation time via the calculation that is made after reducing pixels of [4000 × 4000] actually to [1028 × 1028].

Further, conventionally, the contrast range of a target image to be determined has been manually adjusted. Alternatively, a method of relatively extracting the target image to be determined based on a fixed reference has been employed. However, it has not been strictly performed with the recognition of the frame of the analysis target (for example, a lung field). The width of the permeability inside the remaining area is to be strictly limited to the width at which the cardiac area is recognized, by detecting the cardiac area, and filtering the bone density (cutting a certain fixed range of low permeability) according to the present invention. When detecting the cardiac area, or when being viewed by a person who makes a decision, for example, a doctor, a technician, or the like, this enables more strictly adjusting permeability that is easy to be determined. Further, it is more strictly carried out to adjust proper permeability for evaluating coloring.

Further, according to XP and CY, X-ray transmission is changed in accordance with the body condition in order to reduce exposure, and thus transmittance is often changed depending on lung movement during imaging. Further, according to MRI as well, imaging is made with a certain fixed uneven signal due to a change in magnetic field in a specific direction, or the like. With respect to the foregoing, a change in "density/intensity" of a specific organ can be more precisely measured by matching the calculation of returning the transmittance changed from "back ground" transmittance in the periphery to a fixed form with the transmittance of the specific organ, and correcting a signal value at a certain fixed level by evenly correcting unevenness of an overall change in magnetic field. Further, it is made possible to calculate a more accurate change amount by changing the degree of transmittance from characteristics of each organ via a change in numerical value under an imaging condition, and precisely correcting a change in "density/intensity" of the specific organ.

In the present invention, the average value or a change in intensity, and so forth may be calculated, but the data (intensity) obtained from roentgen rays, CT, or the like, may be subjected to "Gamma correction", and there are some cases where the density may not be reflected correctly. The Gamma correction means processing for correcting chroma and brightness of images or the like displayed on a display. Normally, according to a computer, images are displayed on a display, responding to the input signal, but the displayed brightness and chroma differ therefrom depending on display characteristics. Therefore, Gamma correction is used to correct these errors. The Gamma correction adjusts the relative relationship of signals and color data when being input/output to/from the display, thereby resulting in natural-looking displaying. However, images after Gamma correction are no longer the original image, and thus there are some cases where inconvenience often occurs when performing the image processing according to the present invention. Then, the image after the Gamma correction is subjected to "Gamma inverse correction", that is, the inverse filter corresponding to the Gamma correction value to obtain the image before the Gamma correction. This enables acquiring the image before the Gamma correction, and it becomes possible to appropriately perform image processing according to the present invention.

Further, the present invention is not limited to the Gamma correction, and the image is often subjected to processing after restoring an image that is subjected to the other image processing. The image processing to which the image is subjected is analogized from a change in pixel value in an area where density is invariant during imaging, such as for example, a space outside a human body and so forth; and a function in which a change in pixel value thereof is made to be constant is applied to all pixels. This enables acquiring images each that are similarly close to the original image, and it becomes possible to appropriately perform the image processing according to the present invention.

Further, in the present invention, it is possible to restore individual images from superposed images. Conventionally, "roentgen-ray difference image technology" is known. According to this technique, "past and present roentgen images" of the same patient during medical examination, or the like are superposed on each other, and an area changed from the past to the present, that is, an area estimated as being abnormal is emphasized. This enables detecting a cancer at an early stage, for example. As a superposition method, when a plurality of images are imaged as "the first one, the second one, the third one, the fourth one ...", for example, there is provided a method in which "the first one, the second one, and the third one" are made to be "a superposed image as a first one"; "the second one, the third one, and the fourth one" are made to be "a superposed image as a second one"; and "the third one, the fourth one, the fifth one" are made to be "a superposed image as a third one". As to such superposed images, the pixel value becomes high at a portion where a plurality of images are superposed, and the pixel value becomes low at the portion where the plurality of images are not superposed. Such superposed images often cause blurring of the organ contour, and are thus desirably restored to each of original images thereof. According to the present invention, the contour of the organ can be identified, and thus it becomes possible to restore the original images from the superimposed images.

Further, according to the present invention, it is possible to image the difference in frequency of each area in an organ. That is, according to an organ in cyclic motion, the characteristic of each area can be shown by Fourier-transforming the change value in each area to perform weighting like classifying by colors, or the like, based on the constituent ratio of frequency components. For example, it is possible to identify the frequency component that is made to become a peak in each area, and to classify each area by colors. Further, by identifying a ratio at which each frequency component is occupied within a specific frequency band in each area, it is also possible to classify each area by colors depending on the percentage. Further, when the frequency constituent ratio as a reference is, for example, "50% at 10Hz and 50% at 20Hz" in a specific area, it is also possible to visualize a ratio of departing from the frequency constituent ratio as a reference. As to for example, a heart, this enables grasping at a glance whether to be in correct motion, or to be incorrect motion by displaying a frequency spectrum distribution with classification by colors.

### EXPLANATION OF THE SYMBOLS

1 Basic module
3 Cardiac function analysis section
5 Blood flow analysis section
7 Another blood flow analysis section
9 Fourier analysis section
10 Waveform analysis section
11 Visualization/digitization section
13 Input interface
15 Database
17 Output interface
19 Display

## Claims

1. A diagnostic support program that analyzes images of a human organ and displays analysis results, the program causing a computer to execute a process comprising:
processing of acquiring a plurality of frame images;
processing of calculating a frequency characterizing a state of the organ based on each of the frame images;
processing of calculating a phase difference between a waveform in a previously acquired organ model and a waveform corresponding to the calculated frequency; and
processing of outputting a signal indicating the phase difference.

2. The diagnostic support program according to claim 1,
wherein the organ model is determined by an average value of pixel values within each divided area, by dividing the images of the organ thereinto.

3. The diagnostic support program according to claim 2,
wherein the images of the organ are divided thereinto using a Voronoi tessellation method.

4. The diagnostic support program according to claim 2,
wherein a lung field area is divided thereinto according to a change rate of a lung volume, when the organ is a lung.

5. A diagnostic support program that analyzes images of a human organ and displays analysis results, the program causing a computer to execute a process comprising:
processing of acquiring a plurality of frame images;
processing of extracting pixel values at specific points included in the images of the organ;
processing of calculating a temporal change of the extracted pixel values;
processing of extracting at least one frequency signal from the temporal change of the pixel values; and
processing of outputting the extracted frequency signal as a signal at the specific points.

6. A diagnostic support program that analyzes images of a human organ and displays analysis results, the program causing a computer to execute a process comprising:
processing of acquiring the images of the organ, that are imaged from multiple directions under a plurality of imaging conditions;
processing of extracting pixel values at specific points included in the image of the organ;
processing of calculating a temporal change of the extracted pixel values;
processing of extracting at least one frequency signal from the temporal change of the pixel values;
processing of outputting the extracted frequency signal as a signal at the specific points; and
processing of preparing a cross-sectional diagram of all or part of the organ, using the output signal at the specific points.

7. The diagnostic support program according to claim 5 or 6,
wherein the images of the organ are divided into certain areas including the specific points, using a Voronoi tessellation method.

8. A diagnostic support program that analyzes images of a human organ and displays analysis results, the program causing a computer to execute a process comprising:
processing of acquiring the signal output from the diagnostic support program according to any one of claims 1 to 7;
processing of making AI (Artificial Intelligence) learn the signal indicating a normal organ or the signal indicating an abnormal organ according to the acquired signal; and
processing of recording learning results obtained by the AI.

9. The diagnostic support program according to claim 8, the program causing the computer to execute the process further comprising:
processing of acquiring the plurality of flame images; and
processing of specifying the organ from the acquired flame images, and outputting a ratio of an abnormal value by comparing the specified organ with the learning results.

10. The diagnostic support program according to claim 8, the program causing the computer to execute the process further comprising:
processing of outputting a phase difference between a waveform representing a cyclic motion of the normal organ and a waveform representing a cyclic motion of the abnormal organ.

11. The diagnostic support program according to any one of claims 1 to 8, the program causing the computer to execute the process further comprising:
processing of adding/subtracting a signal in one region exhibiting different permeability therefrom to/from a signal in the other region, according to each of the plurality of flame images.

12. The diagnostic support program according to any one of claims 1 to 8, the program causing the computer to execute the process further comprising:
processing of converting a waveform of a cyclic signal into a trigonometric function; and
processing of outputting a signal indicating the waveform converted into the trigonometric function.

13. A diagnostic support program that analyzes images of a human organ and displays analysis results, the program causing a computer to execute a process comprising:
processing of acquiring a plurality of frame images imaged by applying a cyclic signal to a human body;
processing of Fourier-transforming a change in pixel value in a specific area in each of the frame images;
processing of extracting a spectrum in a fixed band including a spectrum corresponding to a frequency of the signal applied to the human body, out of a spectrum obtained after the Fourier-transforming;
processing of subjecting the spectrum extracted from the fixed band to inverse Fourier transform; and
processing of displaying each of the images after performing the inverse Fourier transform, on a display.

14. A diagnostic support program that analyzes images of a human organ and displays analysis results, the program causing a computer to execute a process comprising:
processing of acquiring a plurality of frame images imaged by applying a cyclic signal to a human body;
processing of calculating a change rate of a pixel value in a specific area in each of the frame images;
processing of extracting only an area for which a tunable rate is within a predetermined fixed range, using the tunable rate that is a value of a ratio of the change rate of the pixel value in the specific area to a cyclic change rate of the signal applied to the human body; and
processing of displaying each of the images including the extracted area, on a display.

15. A diagnostic support program that analyzes images of a human organ and displays analysis results, the program causing a computer to execute a process comprising:
processing of acquiring a plurality of frame images;
processing of calculating a function representing a frequency or a waveform of lung blood pressure, based on each of the frame images;
processing of calculating the function representing the waveform of the lung blood flow, based on the function representing the calculated frequency and waveform of the lung blood flow, and information indicating size of lung blood vessels; and
processing of estimating lung blood pressure from the calculated waveform of the lung blood pressure.

16. The diagnostic support program according to claim 8, the program causing the computer to execute the process further comprising:
processing of acquiring the plurality of frame images; and
processing of specifying the organ and blood flow flowing through the organ from the acquired flame images, and outputting a feature amount of the blood flow in the organ by comparing the specified organ and blood flow with the recorded learning results.

17. The diagnostic support program according to claim 16, the program causing the computer to execute the process further comprising:
processing of outputting a feature amount of blood flow in main blood vessels of the lung, or blood flow in capillaries and peripheral pulmonary vessels of the lung.

18. The diagnostic support program according to claim 16, the program causing the computer to execute the process further comprising:
processing of comparing movement during lung respiration with movement of lung blood flow, and outputting a feature amount indicating a linkage between both the movements.

19. The diagnostic support program according to claim 8, the program causing the computer to execute the process further comprising:
processing of acquiring the plurality of frame images; and
processing of specifying the lung field area from the acquired frame images, comparing a wave indicating movement in the specified lung field area with a reference wave, and outputting a feature amount indicating a linkage between the waves.

20. The diagnostic support program according to claim 19,
wherein the reference wave is a wave indicating a respiratory cycle.

21. The diagnostic support program according to claim 1, the program causing the computer to execute the process further comprising:
processing of calculating a maximum value of pixel value; and
processing of acquiring the waveform, based on a signal after the calculated maximum value.

22. The diagnostic support program according to claim 1,
wherein Fourier-transforming processing is carried out by inputting data having periodicity, and inverse Fourier transform processing is carried out by performing filtering processing by which a specific frequency is extracted.

23. The diagnostic support program according to claim 1,
wherein a plurality of waveforms are superposed, and a phase peak in one cycle of any waveform is detected to calculate a phase difference of any other waveform.

24. The diagnostic support program according to claim 1,
wherein a lung image is divided into a plurality of areas, and an average and a distribution of intensity values in the respective areas are calculated to obtain a correlation with a count value according to lung scintigraphy in between.

25. The diagnostic support program according to claim 1,
wherein a basic waveform is generated by superposing a plurality of original waveforms obtained from the images, respectively; and the original waveforms are subjected to setting of a band width thereof or weighting, while generating a master waveform based on the basic waveform to generate a waveform corresponding to the organ in each of the images.
